# FASCICULE DE BREVET EUROPEEN

(11) **EP 2 935 231 B1**
(45) Date de publication et mention de la délivrance du brevet: **17.05.2017**
(21) Numéro de dépôt: 13818341.3
(22) Date de dépôt: 18.12.2013
(51) Int. Cl.: C07D 309/10

(54) **COMPOSÉS C-XYLOSIDES,COMPOSITIONS ET LEUR UTILISATION POUR DÉPIGMENTER LA PEAU**
C-XYLOSID-VERBINDUNGEN, ZUSAMMENSETZUNGEN UND VERWENDUNG DAVON ZUR DEPIGMENTIERUNG DER HAUT
C-XYLOSIDE COMPOUNDS, COMPOSITIONS AND USE THEREOF TO DEPIGMENT THE SKIN

(30) Priorité: 21.12.2012 FR 1262731
(43) Date de publication de la demande: 28.10.2015
(73) Titulaire: L'OREAL, 75008 Paris (FR)
(72) Inventeur: DALKO, Maria, F-78000 Versailles (FR); MARAT, Xavier, F-75010 Paris (FR); BERNERD, Françoise, F-06100 Nice (FR); COHEN, Catherine, 94500 Champigny sur Marne (FR)
(74) Mandataire: Le Blainvaux Bellegarde, Françoise
(86) Numéro de dépôt international: PCT/FR2013/053160
(87) Numéro de publication internationale: WO 2014/096699

(56) Documents cités:
- EP-A1- 1 774 990
- WO-A1-2010/067036
- BISHT S S ET AL: "Aldol reaction of beta-C-glycosylic ketones: synthesis of C-(E)-cinnamoyl glycosylic compounds as precursors for new biologically active C-glycosides", CARBOHYDRATE RESEARCH, PERGAMON, GB, vol. 343, no. 9, 7 juillet 2008 (2008-07-07), pages 1399-1406, XP022697085, ISSN: 0008-6215, DOI: 10.1016/J.CARRES.2008.04.021 [extrait le 2008-04-23]
- BISHT S S ET AL: "Synthetic studies in butenonyl C-glycosides: Preparation of polyfunctional alkanonyl glycosides and their enzyme inhibitory activity", BIOORGANIC & MEDICINAL CHEMISTRY LETTERS, PERGAMON, ELSEVIER SCIENCE, GB, vol. 19, no. 10, 15 mai 2009 (2009-05-15), pages 2699-2703, XP026085946, ISSN: 0960-894X, DOI: 10.1016/J.BMCL.2009.03.136 [extrait le 2009-03-29]

## Description

La présente invention concerne l'utilisation cosmétique ou pharmaceutique, en particulier dermatologique, d'au moins un composé C-xyloside pour blanchir la peau et pour la dépigmenter ainsi que certains composés nouveaux C-xyloside, les compositions les contenant et leur utilisation pour blanchir la peau et/ou pour la dépigmenter.

La couleur de la peau humaine est fonction de différents facteurs et notamment des saisons de l'année, de la race et du sexe; elle est principalement déterminée par la nature et la concentration de mélanine produite par les mélanocytes. Les mélanocytes sont les cellules spécialisées qui, par l'intermédiaire d'organelles particuliers, les mélanosomes, synthétisent la mélanine. En outre, à différentes périodes de leur vie, certaines personnes voient apparaître sur la peau et plus spécialement sur les mains, des taches plus foncées et/ou plus colorées, conférant à la peau une hétérogénéité. Ces taches sont dues aussi à une concentration importante de mélanine dans les kératinocytes situés à la surface de la peau.

L'utilisation de substances dépigmentantes topiques inoffensives présentant une bonne efficacité est tout particulièrement recherchée en vue de traiter les hyperpigmentations régionales par hyperactivité mélanocytaire telles que les mélasmas idiopathiques, survenant lors de la grossesse ("masque de grossesse" ou chloasma) ou d'une contraception oestro-progestative, les hyperpigmentations localisées par hyperactivité et prolifération mélanocytaire bénigne, telles que les taches pigmentaires séniles dites lentigo actiniques, les hyperpigmentations accidentelles, éventuellement dues à la photosensibilisation ou à la cicatrisation post-lésionnelle. Pour ces dernières (les cicatrisations pouvant aboutir à une cicatrice donnant à la peau un aspect plus blanc), à défaut de pouvoir repigmenter la peau lésée, on achève de dépigmenter les zones de peau normale résiduelle pour donner à l'ensemble de la peau une teinte blanche homogène.

Le mécanisme de formation de la pigmentation de la peau, c'est-à-dire de la formation de la mélanine est particulièrement complexe et fait intervenir schématiquement les principales étapes suivantes :

### Tyrosine ---> Dopa ---> Dopaquinone ---> Dopachrome ---> Mélanine

La tyrosinase (monophénol dihydroxyl phénylalanine : oxygen oxydo-reductase EC 1.14.18.1) est l'enzyme essentielle intervenant dans cette suite de réactions. Elle catalyse notamment la réaction de transformation de la tyrosine en Dopa (dihydroxyphénylalanine) grâce à son activité hydroxylase et la réaction de transformation de la Dopa en dopaquinone grâce à son activité oxydase. Cette tyrosinase n'agit que lorsqu'elle est à l'état de maturation sous l'action de certains facteurs biologiques.

Une substance est reconnue comme dépigmentante si elle agit directement sur la vitalité des mélanocytes épidermiques où se déroule la mélanogénèse, et/ou si elle interfère avec une des étapes de la biosynthèse de la mélanine soit en inhibant une des enzymes impliquées dans la mélanogénèse, soit en s'intercalant comme analogue structural d'un des composés chimiques de la chaîne de synthèse de la mélanine, chaîne qui peut alors être bloquée et ainsi assurer la dépigmentation.

Les substances les plus utilisées en tant que dépigmentants sont plus particulièrement l'hydroquinone et ses dérivés, en particulier ses éthers tels que le monométhyléther et le monoéthyléther d'hydroquinone. Ces composés, bien qu'ils présentent une efficacité certaine, ne sont malheureusement pas exempts d'effets secondaires du fait de leur toxicité, ce qui peut rendre leur emploi délicat, voire dangereux. Cette toxicité provient de ce qu'ils interviennent sur des mécanismes fondamentaux de la mélanogénèse en tuant des cellules qui risquent alors de perturber leur environnement biologique et qui par conséquent obligent la peau à les évacuer en produisant des toxines.

Ainsi, l'hydroquinone est un composé particulièrement irritant et cytotoxique pour le mélanocyte, dont le remplacement, total ou partiel a été envisagé par de nombreux auteurs.

On a ainsi cherché des substances qui n'interviennent pas dans le mécanisme de la mélanogénèse mais qui agissent en amont sur la tyrosinase en empêchant son activation et sont de ce fait beaucoup moins toxiques. On utilise couramment comme inhibiteur de l'activation de la tyrosinase l'acide kojique qui complexe le cuivre présent dans le site actif de cette enzyme. Malheureusement, ce composé est instable en solution, ce qui complique quelque peu la fabrication de la composition.

Des C-glycosides ont déjà été décrits dans l'EP 1774990 pour dépigmenter la peau.

Il subsiste le besoin d'un nouvel agent blanchissant notamment de la peau humaine à action aussi efficace que ceux connus, mais n'ayant pas leurs inconvénients, c'est-à-dire qui soit non irritant, non toxique et/ou non allergisant pour la peau, tout en étant stable dans une composition, ou bien alternativement qui possède une action renforcée de façon à pouvoir être utilisé en quantité plus faible, ce qui diminue considérablement les effets secondaires observés.

A cet égard la Demanderesse a de manière surprenante et inattendue découvert que certains composés C-xylosides présentaient une bonne activité dépigmentante, même à faible concentration, sans faire preuve de cytotoxicité.

De façon plus précise, l'invention a donc pour objet un procédé cosmétique de dépigmentation, d'éclaircissement et/ou de blanchiment de la peau, des poils ou des cheveux comprenant l'application sur la peau, les poils ou les cheveux d'une composition comprenant, dans un milieu physiologiquement acceptable,au moins un composé de formule (I) suivante 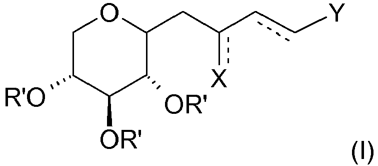 dans laquelle,
- les composés de formule (I) sont des dérivés de xylose
- Y désigne un radical phényle ou un hétérocycle, éventuellement substitués par 1 à 5 groupements (ORₐ)
- X = -OR' ; (=O) ; NR_{b}R_{c} ; NHOR_{d}
- R' désigne :
   * un atome d'hydrogène,
   * un radical alkyle linéaire et saturé en C1-C18,
   *un radical alkyle linéaire et insaturé C2-C18
   * un radical alkyle ramifié, saturé ou insaturé, en C3-C18
   * un radical cyclique saturé ou insaturé en C5 ou C6
   * un radical acyle, linéaire ou ramifié, saturé ou insaturé, en C2-C18, ou cyclique saturé ou insaturé en C5 ou C6.

**Rₐ** désigne :
- un atome d'hydrogène
- un radical alkyle linéaire ou ramifié, en C1-C4, ou un radical hydrocarboné insaturé linéaire ou ramifié en C3-C4
- un radical acyle linéaire ou ramifié en C2-C18 ou alcénylcarbonyle linéaire ou ramifié en C2-C18
- Lorsque Y désigne un radical phényle ou un hétérocycle substitué par 2 à 5 groupements (ORₐ), deux groupements adjacents ORₐ peuvent former ensemble un radical divalent -O-CH₂-O
   sous réserve que lorsque X=OH, le composé ne comporte pas de double liaison éthylénique en position alpha du carbone portant cet OH.

**R_{b}** désigne :
- un atome d'hydrogène
- un radical alkyle linéaire en C2-C18, ou ramifié en C3-C18, ou un radical hydrocarboné insaturé linéaire ou ramifié en C3-C4 ou un radical -CH(Z₁)-CO₂Z₂ dans lequel
   Z₁ désigne un atome d'hydrogène ou un radical alkyle linéaire ou ramifié en C1-C6, ou cyclique saturé ou insaturé en C3-C6, ledit radical étant éventuellement substitué par au moins un groupement choisi parmi =NH, -NH₂, -N(T)₂, =O, -OH, - OT, -SH, -ST, -CO₂T, phényle, phényle substitué par -OH ou -OT, 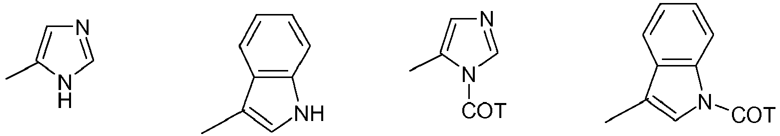 et/ou interrompu par un groupement -NH-, -N-(COT)-, ou -S- avec T désignant un radical alkyle linéaire ou ramifié en C1-C6, ou cyclique en C3-C6.
   et Z₂ désigne un atome d'hydrogène ou un radical alkyle linéaire ou ramifié en C1-C6

**R_{c}** désigne :
- un atome d'hydrogène
- un radical alkyle linéaire en C1-C4, ou ramifié en C3-C4, ou un radical hydrocarboné insaturé linéaire ou ramifié en C3-C4, ledit radical étant éventuellement substitué par un groupement phényle.

**Rd** désigne :
- un atome d'hydrogène
- un radical alkyle linéaire en C1-C18, ou ramifié en C3-C18, ou un radical hydrocarboné insaturé linéaire ou ramifié en C3-C18, ledit radical étant éventuellement substitué par un groupe phényle,
ainsi que leurs sels cosmétiquement acceptables, leurs solvates tels que les hydrates et leurs stéréoisomères,
Au sens de la présente invention, hétérocycle désigne un radical hydrocarboné cyclique 5 à 10 chainons, saturé ou insaturé, y compris aromatique, comprenant au moins un hétéroatome choisi parmi O, S ou N. De préférence, hétérocycle désigne un radical pyridine, pyrimidine, indole et plus préférentiellement pyridine ou indole.

Les composés de formule (I) selon l'invention permettent de dépigmenter et/ou d'éclaircir efficacement la peau d'êtres humains. Ils sont notamment destinés à être appliqués sur la peau d'individus présentant des taches de pigmentation brunâtres, des taches de sénescence, ou sur la peau d'individus désirant combattre l'apparition d'une couleur brunâtre provenant de la mélanogénèse, par exemple à la suite d'une exposition aux rayonnements ultra-violet.

Ils peuvent également permettre de dépigmenter et/ou d'éclaircir les poils, les cils, les cheveux, ainsi que les lèvres et/ou les ongles.

L'invention a donc également pour objet un procédé cosmétique de dépigmentation, d'éclaircissement et/ou de blanchiment de la peau humaine, des poils et /ou des cheveux comprenant l'application sur la peau d'une composition telle que décrite précédemment. Le procédé convient notamment pour éliminer les taches pigmentaires brunâtres et/ou les taches de sénescence, et/ou pour éclaircir la peau brunie.

L'invention a encore pour objet l'utilisation cosmétique d'un composé de formule (I) tel que décrit précédemment comme agent blanchissant et/ou dépigmentant de la peau, des poils, des cils, des cheveux, ainsi que les lèvres et/ou les ongles et de préférence la peau notamment pour éliminer les taches pigmentaires, les taches de sénescence et/ou en tant qu'agents anti-brunissement.

Parmi les composés de formule (I), certains sont connus de l'état de la technique.

Le document " Separation of α,β-anomers of C-glycosides by medium-pressure liquid chromatography" ; Sepu 1988, 6(5), pages 301-3 décrit les composés 2-déoxy-2-(2-(4-méthoxy-phényl)-éthyl)-D-glucose (CAS 121285-89-0) et 2-déoxy-2-(2-(4-méthoxy-phényl)-éthyl)-L-glucose (CAS 121285-90-3).

Le document « Reaction of unsubstituted aldoses with p-méthoxybenzoylméthylenephosphorane » ; Zhurnal Obshchei Khimii 1968, 38(5), pages 1046-8 décrit les composés 2-déoxy-2-(2-oxo-2-(4-méthoxy-phényl)-éthyl)-L-fucose (CAS 20880-40-4), 2-déoxy-2-(2-oxo-2-(4-méthoxy-phényl)-éthyl)-D-xylose (CAS 20869-22-1) et 2-déoxy-2-(2-oxo-2-(4-méthoxy-phényl)-éthyl)-L-xylose (CAS 20869-24-3).

Le document « Interaction of partially screened aldoses with p-méthoxybenzoylméthylenephosphorane" ; Zhurnal Obshchei Khimii 1969, 39(1), pages 119-22 décrit les composés 2-déoxy-2-(2-oxo-2-(4-méthoxy-phényl)-éthyl)-D-maltose (CAS 24461-52-7) et 2-déoxy-2-(2-oxo-2-(4-méthoxy-phényl)-éthyl)-L-maltose (CAS 24461-51-6).

L'invention a donc aussi pour objet les composés de formule (II) à (V) suivantes :

### Composés de formules (II)

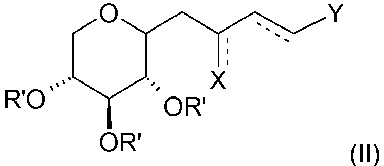

dans laquelle,
- les composés de formule (II) sont des dérivés de xylose
- Y désigne un hétérocycle, éventuellement substitué par 1 à 5 groupements (ORₐ)
- X = -OR' ; (=O) ; NR_{b}R_{c} ; NHOR_{d}
- R' désigne
   * un atome d'hydrogène,
   * un radical alkyle linéaire et saturé en C1-C18,
   * un radical alkyle linéaire et insaturé C2-C18
   * un radical alkyle ramifié, saturé ou insaturé, en C3-C18
   * un radical cyclique saturé ou insaturé en C5 ou C6
   * un radical acyle, linéaire ou ramifié, saturé ou insaturé, en C2-C18, ou cyclique saturé ou insaturé en C5 ou C6.

**Rₐ** désigne :
- un atome d'hydrogène
- un radical alkyle linéaire ou ramifié, en C1-C4, ou un radical hydrocarboné insaturé linéaire ou ramifié en C3-C4
- un radical acyle linéaire ou ramifié en C2-C18 ou alcénylcarbonyle linéaire ou ramifié en C2-C18,

**R_{b}** désigne :
- un atome d'hydrogène
- un radical alkyle linéaire en C2-C18, ou ramifié en C3-C18, ou un radical hydrocarboné insaturé linéaire ou ramifié en C3-C4 ou un radical -CH(Z₁)-CO₂Z₂ dans lequel
   **Z₁** désigne un atome d'hydrogène ou un radical alkyle linéaire ou ramifié en C1-C6, ou cyclique saturé ou insaturé en C3-C6, ledit radical étant éventuellement substitué par au moins un groupement choisi parmi =NH, -NH₂, -N(T)₂, =O, -OH,-OT, -SH, -ST, -CO₂T, phényle, phényle substitué par -OH ou -OT, 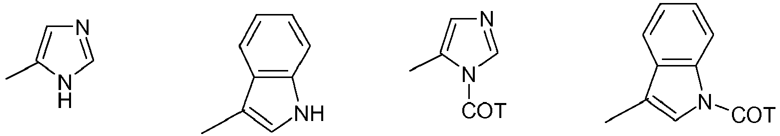 et/ou interrompu par un groupement -NH-, -N-(COT)-, ou -S- avec T désignant un radical alkyle linéaire ou ramifié en C1-C6, ou cyclique en C3-C6.
   et **Z₂** désigne un atome d'hydrogène ou un radical alkyle linéaire ou ramifié en C1-C6

**Rc** désigne :
- un atome d'hydrogène
- un radical alkyle linéaire en C1-C4, ou ramifié en C3-C4, ou un radical hydrocarboné insaturé linéaire ou ramifié en C3-C4, ledit radical étant éventuellement substitué par un groupement phényle.

**Rd** désigne :
- un atome d'hydrogène
- un radical alkyle linéaire en C1-C18, ou ramifié en C3-C18, ou un radical hydrocarboné insaturé linéaire ou ramifié en C3-C18, ledit radical étant éventuellement substitué par un groupe phényle,
ainsi que leurs sels cosmétiquement acceptables, leurs solvates tels que les hydrates et leurs stéréoisomères,
Au sens de la présente invention, hétérocycle désigne un radical hydrocarboné cyclique 5 à 10 chainons, saturé ou insaturé, y compris aromatique, comprenant au moins un hétéroatome choisi parmi O, S ou N. De préférence, hétérocycle désigne un radical pyridine, pyrimidine, indole et plus préférentiellement pyridine ou indole.

### Composés nouveaux de formule (III) :

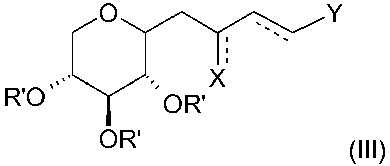

dans laquelle,
- les composés de formule (III) sont des dérivés de xylose
- Y désigne un radical phényle éventuellement substitué par 1 à 5 groupements (ORₐ)
- X = -OR" ; NR_{b}R_{c} ; NHOR_{d}
- R' désigne :
   * un atome d'hydrogène,
   * un radical alkyle linéaire ou ramifié, saturé ou insaturé en C1-C18, , ou cyclique saturé ou insaturé en C5 ou C6
   * un radical acyle linéaire ou ramifié, saturé ou insaturé en C1-C18, ou cyclique saturé ou insaturé en C5 ou C6.
- R" désigne :
   * un radical alkyle linéaire ou ramifié, saturé ou insaturé en C1-C18, , ou cyclique saturé ou insaturé en C5 ou C6
   * un radical acyle linéaire ou ramifié, saturé ou insaturé en C1-C18, , ou cyclique saturé ou insaturé en C5 ou C6.

**Rₐ** désigne :
- un atome d'hydrogène
- un radical alkyle linéaire ou ramifié en C1-C4, ou un radical hydrocarboné insaturé linéaire ou ramifié en C3-C4
- un radical acyle linéaire ou ramifié en C1-C18 ou alcénylcarbonyle linéaire ou ramifié en C2-C18
Lorsque Y désigne un radical phényle substitué par 2 à 5 groupements (ORₐ), deux groupements adjacents ORₐ peuvent former ensemble un radical divalent-O-CH₂-O,

**R_{b}** désigne :
- un atome d'hydrogène
- un radical alkyle linéaire en C2-C18, ou ramifié en C3-C18, ou un radical hydrocarboné insaturé linéaire ou ramifié en C3-C4 ou un radical -CH(Z₁)-CO₂Z₂ dans lequel
   Z₁ désigne un atome d'hydrogène ou un radical alkyle linéaire ou ramifié en C1-C6, ou cyclique saturé ou insaturé en C3-C6, ledit radical étant éventuellement substitué par au moins un groupement choisi parmi =NH, -NH₂, -N(T)₂, =O, -OH, - OT, -SH, -ST, -CO₂T, phényle, phényle substitué par -OH ou -OT, 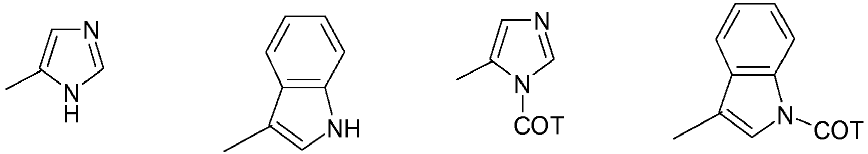 et/ou interrompu par un groupement -NH-, -N-(COT)-, ou -S- avec T désignant un radical alkyle linéaire ou ramifié en C1-C6, ou cyclique en C3-C6.
   et Z₂ désigne un atome d'hydrogène ou un radical alkyle linéaire ou ramifié en C1-C6

**R_{c}** désigne :
- un atome d'hydrogène
- un radical alkyle linéaire en C1-C4, ou ramifié en C3-C4, ou un radical hydrocarboné insaturé linéaire ou ramifié en C3-C4, ledit radical étant éventuellement substitué par un groupement phényle.

**Rd** désigne :
- un atome d'hydrogène
- un radical alkyle linéaire en C1-C18, ou ramifié en C3-C18, ou un radical hydrocarboné insaturé linéaire ou ramifié en C3-C18, ledit radical étant éventuellement substitué par un groupe phényle
ainsi que leurs sels cosmétiquement acceptables, leurs solvates tels que les hydrates, et leurs stéréoisomères.

### Composés nouveaux de formule (IV):

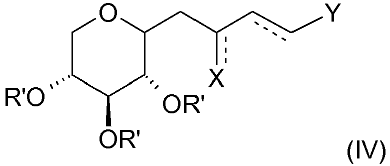

dans laquelle,
- les composés de formule (IV) sont des dérivés de xylose
- Y désigne un radical phényle éventuellement substitué par 1 à 5 groupements (ORₐ)
- X = -OH ; (=O)
- R' désigne :
   * un radical alkyle linéaire ou ramifié, saturé ou insaturé en C1-C18, , ou cyclique saturé ou insaturé en C5 ou C6
   * un radical acyle linéaire ou ramifié, saturé ou insaturé en C1-C18, ou cyclique saturé ou insaturé en C5 ou C6.

**Rₐ** désigne :
- un atome d'hydrogène
- un radical alkyle linéaire ou ramifié en C1-C4, ou un radical hydrocarboné insaturé linéaire ou ramifié en C3-C4
- un radical acyle linéaire ou ramifié en C1-C18 ou alcénylcarbonyle linéaire ou ramifié en C2-C18
Lorsque Y désigne un radical phényle substitué par 2 à 5 groupements (ORₐ), deux groupements adjacents ORₐ peuvent former ensemble un radical divalent-O-CH₂-O
sous réserve que lorsque X=OH, le composé ne comporte pas de double liaison éthylénique en position alpha du carbone portant cet OH.

**R_{b}** désigne :
- un atome d'hydrogène
- un radical alkyle linéaire en C2-C18, ou ramifié en C3-C18, ou un radical hydrocarboné insaturé, linéaire ou ramifié, en C3-C4 ou un radical -CH(Z₁)-CO₂Z₂ dans lequel
   **Z₁** désigne un atome d'hydrogène ou un radical alkyle linéaire ou ramifié en C1-C6, ou cyclique saturé ou insaturé en C3-C6, ledit radical étant éventuellement substitué par au moins un groupement choisi parmi =NH, -NH₂, -N(T)₂, =O, -OH,-OT, -SH, -ST, -CO₂T, phényle, phényle substitué par -OH ou -OT, 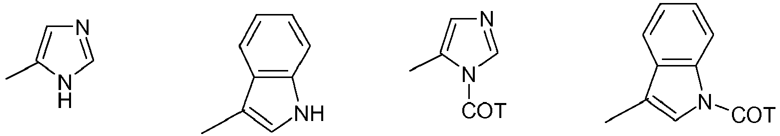 et/ou interrompu par un groupement -NH-, -N-(COT)-, ou -S- avec T désignant un radical alkyle linéaire ou ramifié en C1-C6, ou cyclique en C3-C6.
   et **Z₂** désigne un atome d'hydrogène ou un radical alkyle linéaire ou ramifié en C1-C6

**R_{c}** désigne :
- un atome d'hydrogène
- un radical alkyle linéaire en C1-C4, ou ramifié en C3-C4, ou un radical hydrocarboné insaturé linéaire ou ramifié en C3-C4 , ledit radical étant éventuellement substitué par un groupement phényle.

**Rd** désigne :
- un atome d'hydrogène
- un radical alkyle linéaire en C1-C18, ou ramifié en C3-C18, ou un radical hydrocarboné insaturé linéaire ou ramifié en C3-C18, ledit radical étant éventuellement substitué par un groupe phényle
ainsi que leurs sels cosmétiquement acceptables, leurs solvates tels que les hydrates, et leurs stéréoisomères,
à l'exception des composés suivants :

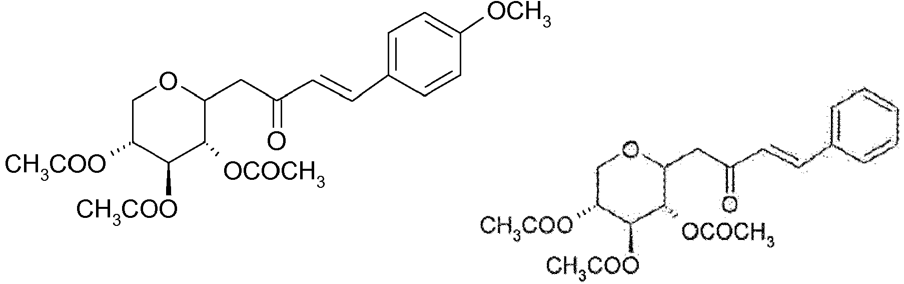

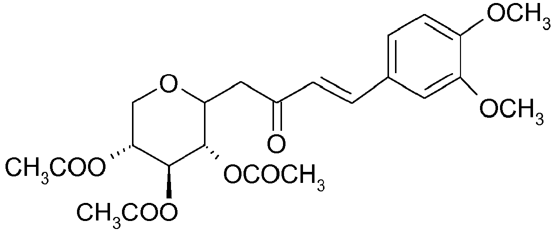

### Composés nouveaux de formule (V) :

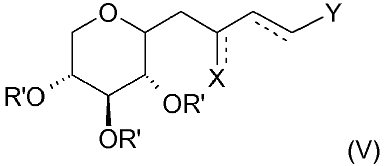

dans laquelle,
- les composés de formule (V) sont des dérivés de xylose
- Y désigne un radical phényle substitué par 1 à 5 groupements (ORₐ)
- X = -OH ; (=O)
- R' désigne un atome d'hydrogène

**Rₐ** désigne
- un radical alkyle linéaire ou ramifié en C1-C4, ou un radical hydrocarboné insaturé linéaire ou ramifié en C3-C4
- un radical acyle linéaire en C1-C18 ou ramifié en C3-C18 ou alcénylcarbonyle linéaire ou ramifié en C2-C18
sous réserve que lorsque X=OH, le composé ne comporte pas de double liaison éthylénique en position alpha du carbone portant cet OH.

Lorsque Y désigne un radical phényle substitué par 2 à 5 groupements (ORₐ), deux groupements adjacents ORₐ peuvent former ensemble un radical divalent-O-CH₂-O

**R_{b}** désigne :
- un atome d'hydrogène
- un radical alkyle linéaire en C2-C18, ou ramifié en C3-C18, ou un radical hydrocarboné insaturé linéaire ou ramifié en C3-C4 ou un radical -CH(Z₁)-CO₂Z₂ dans lequel
   Z₁ désigne un atome d'hydrogène ou un radical alkyle linéaire ou ramifié en C1-C6, ou cyclique saturé ou insaturé en C3-C6, ledit radical étant éventuellement substitué par au moins un groupement choisi parmi =NH, -NH₂, -N(T)₂, =O, -OH, - OT, -SH, -ST, -CO₂T, phényle, phényle substitué par -OH ou -OT, 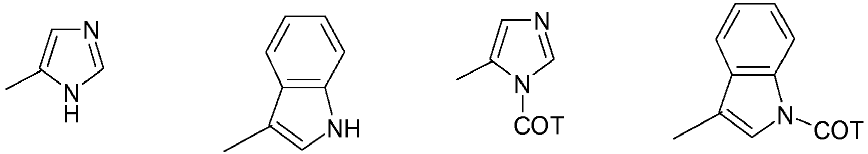 et/ou interrompu par un groupement -NH-, -N-(COT)-, ou -S- avec T désignant un radical alkyle linéaire ou ramifié en C1-C6, ou cyclique en C3-C6.
   et Z₂ désigne un atome d'hydrogène ou un radical alkyle linéaire ou ramifié en C1-C6

**R_{c}** désigne :
- un atome d'hydrogène
- un radical alkyle linéaire en C1-C4, ou ramifié en C3-C4, ou un radical hydrocarboné insaturé linéaire ou ramifié en C3-C4, ledit radical étant éventuellement substitué par un groupement phényle.

**R_{d}** désigne :
- un atome d'hydrogène
- un radical alkyle linéaire en C1-C18, ou ramifié en C3-C18, ou un radical hydrocarboné insaturé linéaire ou ramifié en C3-C18, ledit radical étant éventuellement substitué par un groupe phényle,
ainsi que leurs sels cosmétiquement acceptables, leurs solvates tels que les hydrates, et leurs stéréoisomères,
à l'exception du composé suivant :

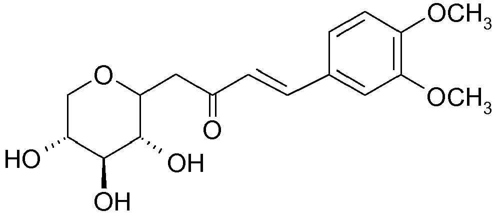

L'invention a aussi pour objet des compositions notamment cosmétiques comprenant notamment dans un milieu physiologiquement acceptable au moins un composés choisis parmi ceux de formule (II) à (V)

Le terme alkyle, dans le cadre de la présente invention, signifie une chaîne hydrocarbonée saturée ou insaturée. Parmi les groupes alkyle convenant à la mise en oeuvre de l'invention, on peut notamment citer les groupes méthyle, éthyle, isopropyle, n-propyle, n-butyle, t-butyle, isobutyle, sec-butyle, pentyle, n-hexyle, n-heptyle, n-octyle, n-nonyle, n-décyle, n-dodécyle, n-tertradécyle, n-hexadécyle, n-heptadécyle, n-octadécyle, allyle, oléyle,
Le terme radical alkyle cyclique, dans le cadre de la présente invention, désigne un radical hydrocarboné cyclique saturé ou cycloalkyle, ou un radical hydrocarboné cyclique insaturé ou cyloalcènyle. Parmi les radicaux alkyles cycliques convenant à la mise en oeuvre de l'invention, on peut notamment citer les groupes cyclopentyle, cyclohexyle, cyclopentènyle, cyclohexènyle.

Les sels acceptables pour l'usage non thérapeutique des composés décrits dans la présente invention comprennent des sels non toxiques conventionnels desdits composés tels que ceux formés à partir d'acides organiques ou inorganiques. A titre d'exemple, on peut citer les sels d'acides minéraux, tels que l'acide sulfurique, l'acide chlorhydrique, l'acide bromhydrique, , l'acide phosphorique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, , l'acide citrique et l'acide tartrique, l'acide méthanesulfonique, l'acide succinique.

Lorsque les composés de formule (I) à (V) comportent un groupe acide, la neutralisation du ou des groupes acides peut être effectuée par une base minérale, telle que LiOH, NaOH, KOH, Ca(OH)₂, NH₄OH, Mg(OH)₂ ou Zn(OH)₂; ou par une base organique telle qu'une alkylamine primaire, secondaire ou tertiaire, par exemple la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine, la diméthylamino-2-propanol, le 2-amino-2-(hydroxyméthyl)-1,3-propanediol. On peut encore citer la lysine ou la 3-(diméthylamino)propylamine.

Les solvates acceptables pour l'usage non thérapeutique des composés décrits dans la présente invention comprennent des solvates conventionnels tels que ceux formés lors de la dernière étape de préparation desdits composés du fait de la présence de solvants. A titre d'exemple, on peut citer les solvates dus à la présence d'eau ou d'alcools linéaires ou ramifiés comme l'éthanol ou l'isopropanol.

Les composés de formule (I) préférés sont ceux pour lesquels :
- les composés de formule (I) sont des dérivés de xylose
- Y désigne un radical phényle ou un hétérocycle, éventuellement substitués par 1 à 5 groupements (ORₐ)
- X = -OR' ; (=O) ; NR_{b}R_{c} ; NHOR_{d}
- R' désigne :
   * un atome d'hydrogène,
   * un radical alkyle linéaire ou ramifié, saturé ou insaturé en C1-C12, ou cyclique saturé en C5 ou C6
   * un radical acyle linéaire ou ramifié, saturé ou insaturé en C1-C6, ou cyclique saturé en C5 ou C6.

**Rₐ** désigne :
- un atome d'hydrogène
- un radical alkyle linéaire ou ramifié, en C1-C4,
- un radical acyle linéaire ou ramifié en C1-C6
Lorsque Y désigne un radical phényle ou un hétérocycle substitué par 2 à 5 groupements (ORₐ), deux groupements adjacents ORₐ peuvent former ensemble un radical divalent -O-CH₂-O
sous réserve que lorsque X=OH, le composé ne comporte pas de double liaison éthylénique en position alpha du carbone portant cet OH.

**R_{b}** désigne :
- un atome d'hydrogène
- un radical alkyle linéaire en C2-C12, ou ramifié en C3-C12, ou un radical-CH(Z₁₎-CO₂Z₂ dans lequel
   Z₁ désigne un atome d'hydrogène ou un radical alkyle linéaire ou ramifié en C1-C6, ou cyclique saturé ou insaturé en C3-C6, ledit radical étant éventuellement substitué par au moins un groupement choisi parmi =NH, -NH₂, -N(T)₂, =O, -OH,-OT, -SH, -ST, -CO₂T, phényle, phényle substitué par -OH ou -OT, 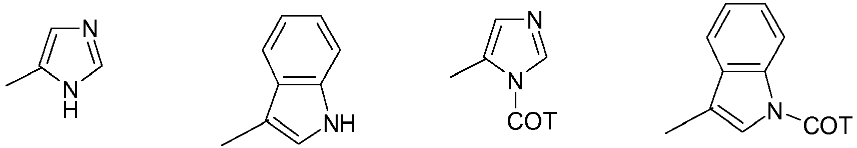 et/ou interrompu par un groupement -NH-, -N-(COT)-, ou -S- avec T désignant un radical alkyle linéaire en C1-C6, cyclique en C5-C6.
   et Z₂ désigne un atome d'hydrogène ou un radical alkyle linéaire ou ramifié en C1-C6

**R_{c}** désigne :
- un atome d'hydrogène
- un radical alkyle linéaire en C1-C4, ou ramifié en C3-C4, ou un radical hydrocarboné insaturé linéaire ou ramifié en C3-C4, ledit radical étant éventuellement substitué par un groupement phényle.

**Rd** désigne :
- un atome d'hydrogène
- un radical alkyle linéaire en C1-C12, ou ramifié en C3-C12, ledit radical étant éventuellement substitué par un groupe phényle,
ainsi que leurs sels cosmétiquement acceptables, leurs solvates tels que les hydrates et leurs stéréoisomères,

Au sens de la présente invention, hétérocycle désigne un radical hydrocarboné cyclique 5 à 10 chainons, saturé ou insaturé, y compris aromatique, comprenant au moins un hétéroatome choisi parmi O, S ou N. De préférence, hétérocycle désigne un radical pyridine, pyrimidine, indole et plus préférentiellement pyridine ou indole.

Les composés de formule (I) particulièrement préférés sont ceux pour lesquels :
- les composés de formule (I) sont des dérivés de xylose
- Y désigne un radical phényle ou un hétérocycle, éventuellement substitués par 1 à 3 groupements (ORₐ)
- X = -OR' ; (=O) ; NR_{b}R_{c} ; NHOR_{d}
- R' désigne :
   * un atome d'hydrogène,
   * un radical alkyle linéaire ou ramifié, saturé ou insaturé en C1-C4,
   * un radical acyle linéaire ou ramifié en C1-C6.

**Rₐ** désigne :
- un atome d'hydrogène
- un radical alkyle linéaire ou ramifié, en C1-C4,
- un radical acyle linéaire ou ramifié en C1-C6
Lorsque Y désigne un radical phényle ou un hétérocycle substitué par 2 ou 3 groupements (ORₐ), deux groupements adjacents ORₐ peuvent former ensemble un radical divalent -O-CH₂-O
sous réserve que lorsque X=OH, le composé ne comporte pas de double liaison éthylénique en position alpha du carbone portant cet OH.

**R_{b}** désigne :
- un atome d'hydrogène
- un radical alkyle linéaire en C2-C8, ou ramifié en C3-C8, ou un radical-CH(Z₁₎-CO₂Z₂ dans lequel
Z₁ désigne un atome d'hydrogène ou un radical alkyle linéaire ou ramifié en C1-C6, ou cyclique saturé ou insaturé en C3-C6, ledit radical étant éventuellement substitué par au moins un groupement choisi parmi =NH, -NH₂, -N(T)₂, =O, -OH, - OT, -SH, -ST, -CO₂T, phényle, phényle substitué par -OH ou -OT, 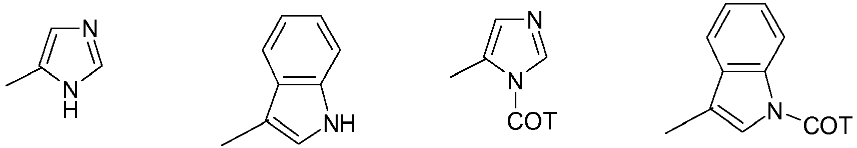 et/ou interrompu par un groupement -NH-, -N-(COT)-, ou -S- avec T désignant un radical alkyle linéaire en C1-C6, cyclique en C5-C6.
et Z₂ désigne un atome d'hydrogène ou un radical alkyle linéaire ou ramifié en C1-C6

**R_{c}** désigne :
- un atome d'hydrogène
- un radical alkyle linéaire en C1-C4, ou ramifié en C3-C4, éventuellement substitué par un groupement phényle.

**Rd** désigne :
- un atome d'hydrogène
- un radical alkyle linéaire en C1-C4, ou ramifié en C3-C4, ledit radical étant éventuellement substitué par un groupe phényle,
ainsi que leurs sels cosmétiquement acceptables, leurs solvates tels que les hydrates et leurs stéréoisomères,

Au sens de la présente invention, hétérocycle désigne un radical hydrocarboné cyclique 5 à 10 chainons, saturé ou insaturé, y compris aromatique, comprenant au moins un hétéroatome choisi parmi O, S ou N. De préférence, hétérocycle désigne un radical pyridine, pyrimidine, indole et plus préférentiellement pyridine ou indole.

Les composés de formule (I) plus particulièrement préférés sont les suivants :
**Composé 1.** : (3R,4S,5R)-2-[2-hydroxy-4-(4-hydroxy-3-methoxyphenyl)butyl]tetrahydro-2H-pyran-3,4,5-triol 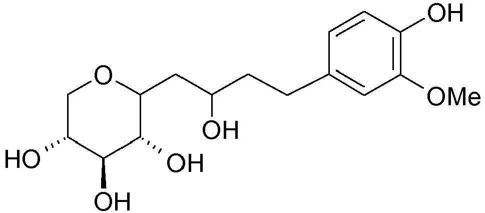
**Composé 2.** 4-(4-hydroxy-3-methoxyphenyl)-1-[(3R,4S,5R)-3,4,5-trihydroxytetrahydro-2H-pyran-2-yl]butan-2-one 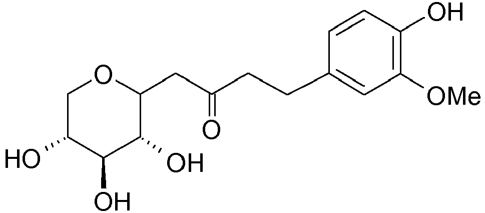
**Composé 3.** (3R,4S,5R)-2-[2-hydroxy-4-(4-hydroxy-phenyl)butyl]tetrahydro-2H-pyran-3,4,5-triol 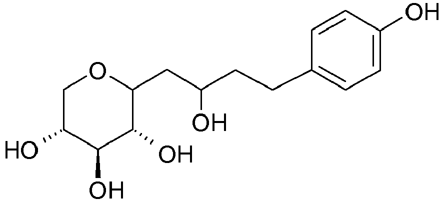
**Composé 4.** 4-(4-hydroxy-phenyl)-1-[(3R,4S,5R)-3,4,5-trihydroxytetrahydro-2H-pyran-2-yl]butan-2-one 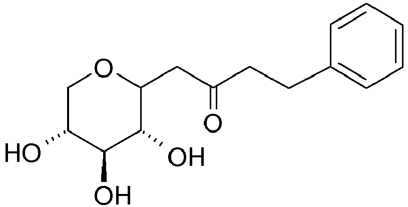
**Composé 5.** (3R,4S,5R)-2-[2-(benzylamino)-4-(4-hydroxy-3-methoxyphenyl)butyl]tetrahydro-2H-pyran-3,4,5-triol 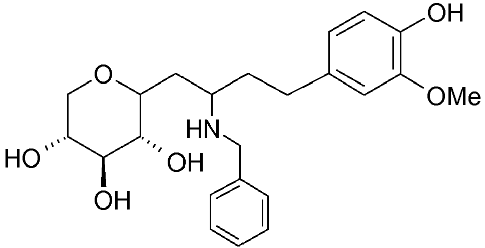
**Composé 6.** ethyl {[3-(4-hydroxy-3-methoxyphenyl)-1-{[(3R,4S,5R)-3,4,5-trihydroxytetrahydro-2H-pyran-2-yl]methyl}propyl]amino}(phenyl)acetate 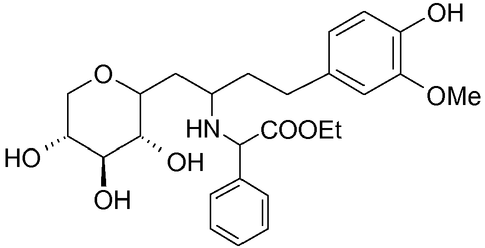
**Composé 7.** (3E)-4-phenyl-1-[(3R,4S,5R)-3,4,5-trihydroxytetrahydro-2H-pyran-2-yl]but-3-en-2-one 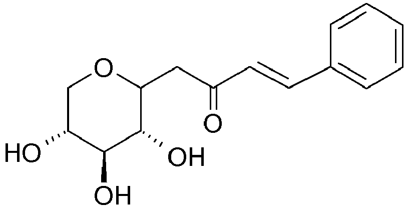
**Composé 8.** (3E)-4-(4-hydroxy-3,5-dimethoxyphenyl)-1-[(3R,4S,5R)-3,4,5-trihydroxytetrahydro-2H-pyran-2-yl]but-3-en-2-one 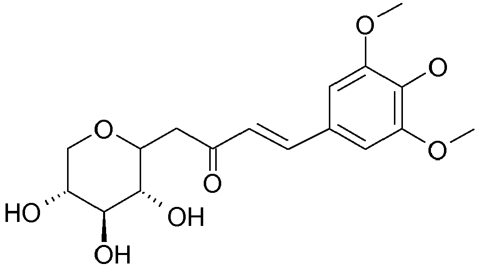 <
**Composé 9.** (3R,4S,5R)-2-[2-hydroxy-4-(2-hydroxyphenyl)butyl]tetrahydro-2H-pyran-3,4,5-triol 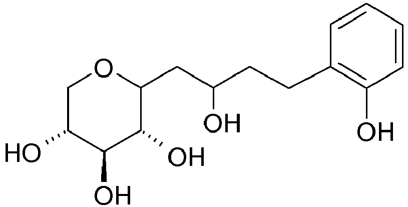
**Composé 10** : (3R,4S,5R)-2-[2-hydroxy-4-(3-hydroxy-4-methoxyphenyl)butyl]tetrahydro-2H-pyran-3,4,5-triol 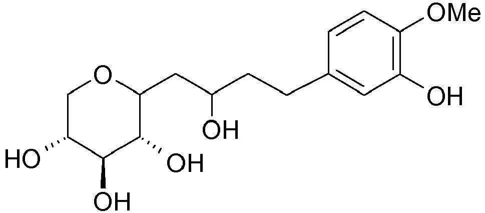
**Composé 11.** (3R,4S,5R)-2-[2-hydroxy-4-(2,4-di-hydroxy-phenyl)butyl]tetrahydro-2H-pyran-3,4,5-triol 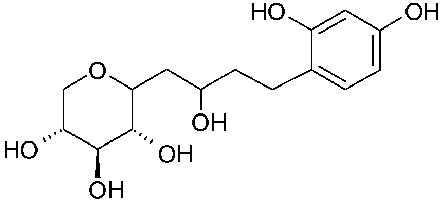
**Composé 12.** (3R,4S,5R)-2-[2-hydroxy-4-(3-ethoxy-4-hydroxy-phenyl)butyl]tetrahydro-2H-pyran-3,4,5-triol 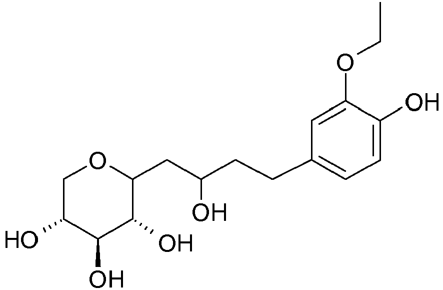
**Composé 13.** 5,9-anhydro-1,2,4-trideoxy-1-pyridin-3-yl-D-xylo-nonitol 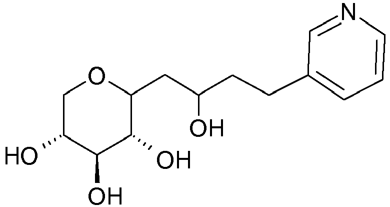
**Composé 14.** (3R,4S,5R)-2-[(2E)-2-(methoxyimino)-4-phenylbutyl]tetrahydro-2H-pyran-3,4,5-triol 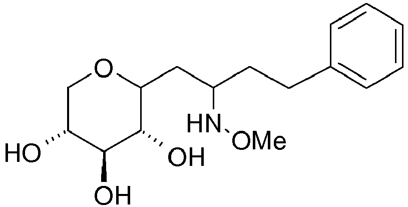
**Composé 15.** 5,9-anhydro-1,2,4-trideoxy-7-O-pentanoyl-1-phenyl-D-xylo-non-3-ulose 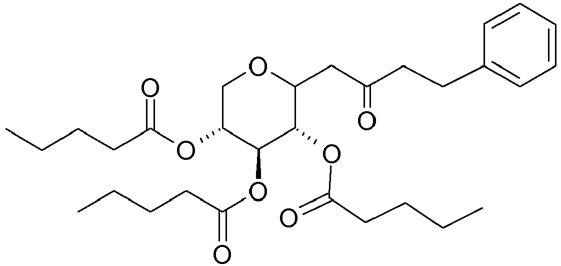
**Composé 16.** 5,9-anhydro-1,2,4-trideoxy-1-(3,4,5-trimethoxyphenyl)-D-xylononitol

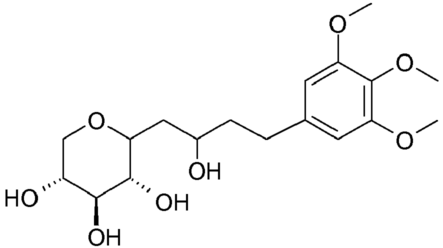

Les composés 5, 6, 10, 11, 12, 13, 14, 15 et 16 sont des composés nouveaux qui constituent un autre objet de l'invention.

La présente invention a également pour objet des compositions notamment cosmétiques comprenant notamment dans un milieu physiologiquement acceptable au moins un composé choisi parmi les composés 5, 6, 10, 11, 12, 13, 14, 15 et 16.

Le composé 13 est le composé préféré de formule (II).

Les composés 5, 6 et 14 sont les composés préférés de formule (III).

Le composé 15 est le composé préféré de formule (IV).

Le composé 16 est le composé préféré de formule (V).

Les composés de formule (I) peuvent être synthétisés selon le mode opératoire général figurant dans la demande EP 2376510.

### a.) Procédé de synthèse général pour les composés nouveaux

### Procédé de synthèse générale pour les composés de formule (II)

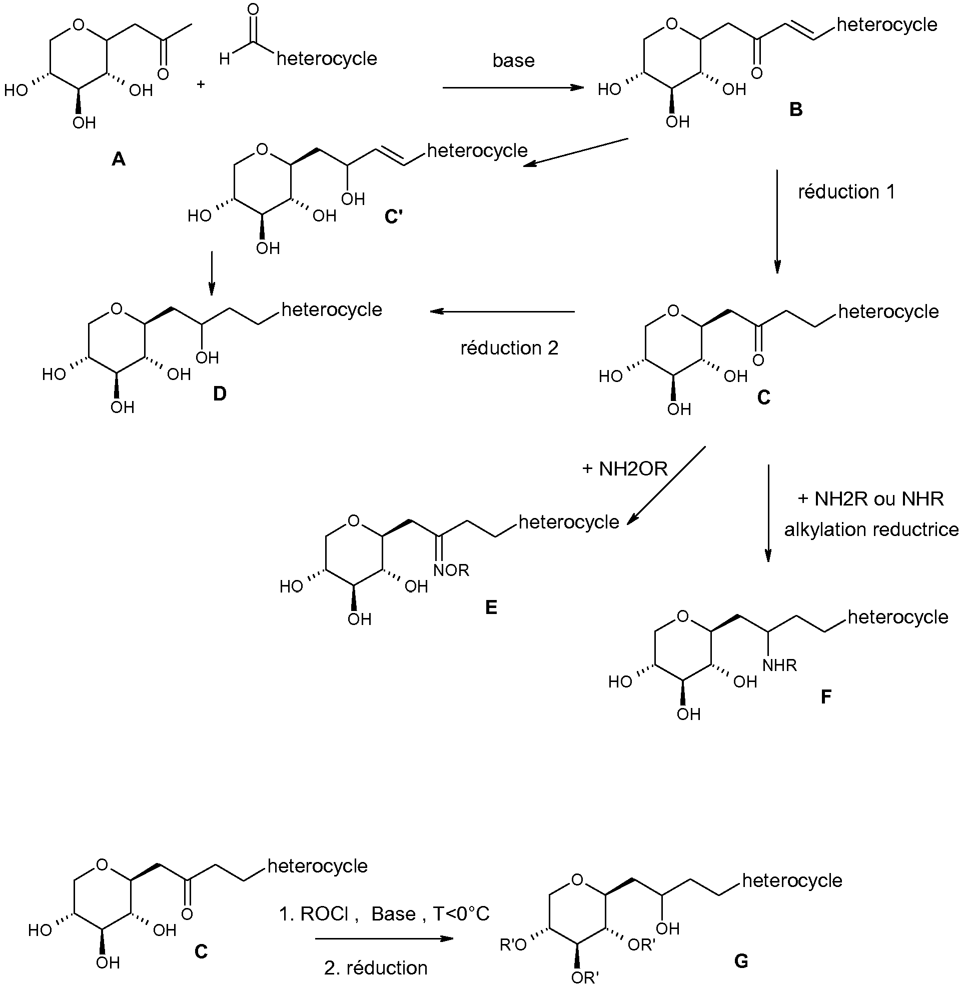

Le C-glycoside A dont la synthèse est décrite dans la demande EP1345919, est mis à réagir avec 1 à 2 éq dealdehyde, en présence d'une base organique (ex . : MeONa) dans un solvant polaire compatible avec cette base (ex. : MeOH). Le milieu réactionnel est agité 1-20h à température ambiante pour former le produit de l'aldolisation B.

Le produit B est ensuite soumis à 2 réductions successives. La première est la réduction de la double liaison par hydrogénation catalytique (ex. : Pd/C / H2) pour obtenir C, et la deuxième est la réduction du carbonyle par un hydrure (ex. : NaBH4) pour obtenir D. On peut également obtenir D en une seule étape à partir de B en combinant les deux étapes de réduction (ex. : Ru/C/H2).

Les composés E peuvent d'être obtenus soit par une simple reaction d'une alkoxyamine NH2-OR avec le composé C dans un solvant tel que l'acetonitrile par exemple; Le composé F peut être obtenu par une alkylation réductrice classique

Les composés G peuvent être obtenus par acylation sélective à basse température d'une des fonction OH du cycle du composé C , suivie par une réduction de carbonyle par un hydrure (ex. : NaBH4).

De manière alternative le composé B peut être soumis en premier lieu à une réduction de la fonction cétone pour conduire à C', puis à une réduction de la double liaison de C' pour fournir les composés D.

### Procédé de synthèse générale pour les composés de formule (III, IV, V)

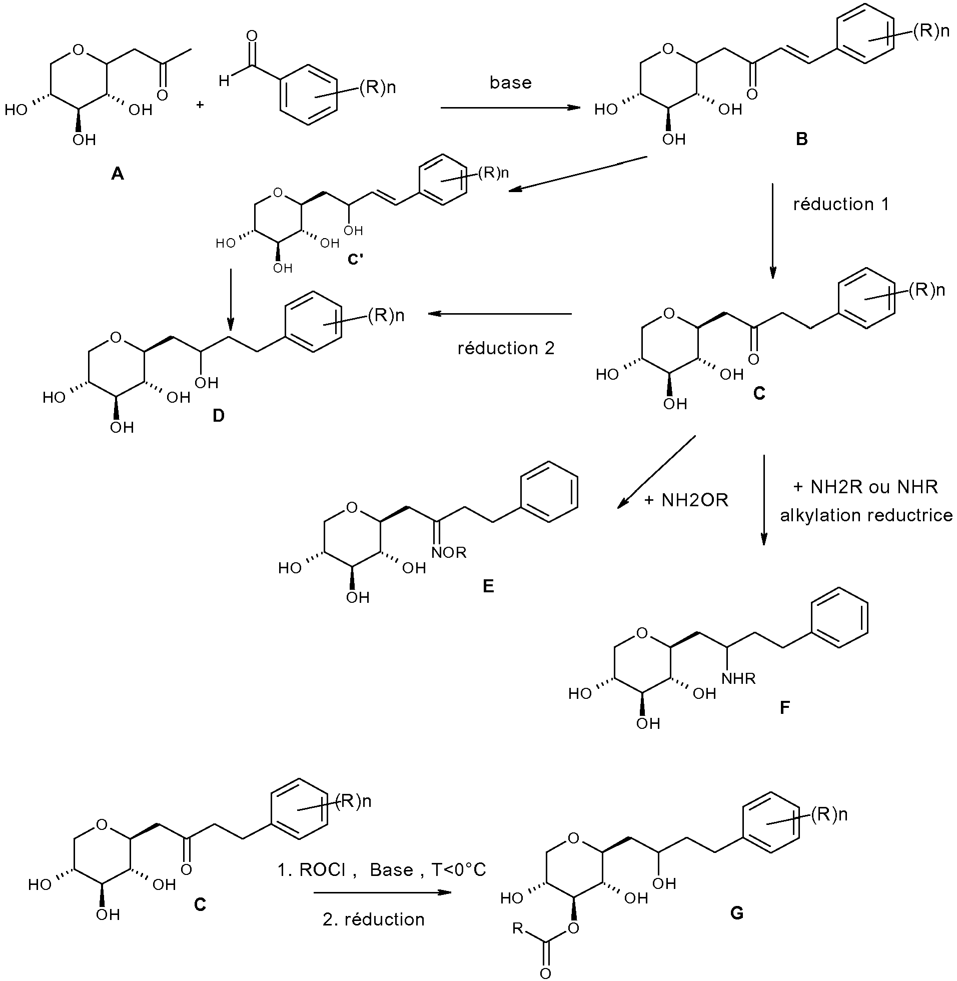

Un autre objet de l'invention concerne une composition qui comprend, dans un milieu physiologiquement acceptable, au moins un dérivé C-xyloside répondant à la formule (I) telle que définie ci-dessus. En particulier la composition est adaptée à une application topique sur la peau. Le milieu physiologiquement acceptable sera préférentiellement un milieu cosmétiquement ou dermatologiquement acceptable, c'est-à-dire sans odeur, couleur ou aspect désagréable, et qui ne génère pas de picotement, tiraillement ou rougeur inacceptable pour l'utilisateur.

Par milieu physiologiquement acceptable, on comprend un milieu compatible avec les matières kératiniques d'êtres humains comme la peau, les muqueuses, les ongles, les poils, les cils, le cuir chevelu et/ou les cheveux.

La composition selon l'invention peut être destinée à une application cosmétique ou pharmaceutique, particulièrement dermatologique. De préférence la composition selon l'invention est destinée à une application cosmétique.

La quantité de composés de formule (I) utilisable dans le cadre de l'invention dépend bien évidemment de l'effet recherché.

A titre d'exemple, cette quantité peut aller par exemple de 0,001% à 10% en poids, de préférence de 0,01% à 5% en poids, notamment de 0,1 à 3% en poids, par rapport au poids total de la composition, notamment de 0,1 à 2%.

La composition peut alors comprendre tous les constituants usuellement employés dans l'application envisagée.

On peut notamment citer l'eau, les solvants, les huiles d'origine minérale, animale et/ou végétale, les cires, les pigments, les charges, les tensioactifs, les actifs cosmétiques ou dermatologiques, les filtres UV, les polymères, les gélifiants, les conservateurs.

Bien entendu l'homme du métier veillera à choisir ce ou ces éventuels composés complémentaires, et/ou leur quantité, de manière telle que les propriétés avantageuses des composés selon l'invention ne soient pas, ou substantiellement pas, altérées par l'adjonction envisagée.

La composition selon l'invention peut se présenter sous toutes les formes galéniques normalement utilisées dans les domaines cosmétique et dermatologique; elle peut être notamment sous forme d'une solution aqueuse, hydroalcoolique, éventuellement gélifiée, d'une dispersion du type lotion éventuellement biphasée, d'une émulsion huile-dans-eau ou eau-dans-huile ou multiple, d'un gel aqueux, d'une dispersion d'huile dans une phase aqueuse à l'aide de sphérules, ces sphérules pouvant être des nanoparticules polymériques telles que les nanosphères et les nanocapsules ou, mieux, des vésicules lipidiques de type ionique et/ou non-ionique.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les éventuels coémulsionnants utilisés dans la composition sous forme d'émulsion sont choisis parmi ceux classiquement utilisés dans le domaine considéré. L'émulsionnant et le coémulsionnant sont présents, dans la composition, en une proportion pouvant aller de 0,3 à 30 % en poids, et de préférence de 0,5 à 20 % en poids par rapport au poids total de la composition.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin et/ou comme produit de maquillage.

Cette composition peut constituer une crème de nettoyage, de protection, de traitement ou de soin pour le visage, pour les mains, pour les pieds, pour les grands plis anatomiques ou pour le corps (par exemple crèmes de jour, crèmes de nuit, crèmes démaquillantes, crèmes de fond de teint, crèmes anti-solaires); un fond de teint fluide, un lait de démaquillage, un lait corporel de protection ou de soin, un lait anti-solaire; une lotion, gel ou mousse pour le soin de la peau, comme une lotion de nettoyage.

Dans un aspect avantageux de l'invention, les compositions utilisées peuvent comporter en plus au moins un agent desquamant, et/ou au moins un agent apaisant, et/ou au moins agent photoprotecteur organique et/ou au moins un agent photoprotecteur inorganique.

Par "agent desquamant", on entend tout composé capable d'agir :
- soit directement sur la desquamation en favorisant l'exfoliation, tel que les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'urée ; l'acide gentisique ; les oligofucoses ; l'acide cinnamique ; l'extrait de Saphora japonica ; le resvératrol ;
- soit sur les enzymes impliquées dans la desquamation ou la dégradation des cornéodesmosomes, les glycosidases, la stratum corneum chymotryptic enzym (SCCE) voire d'autres protéases (trypsine, chymotrypsine-like). On peut citer les agents chélatant des sels minéraux : l'EDTA ; l'acide N-acyl-N,N',N' éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le miel ; les dérivés de sucre tels que l'O-octanoyl-6-D-maltose et la N-acétyl glucosamine.

Comme agents apaisants utilisables dans la composition selon l'invention, on peut citer : les triterpènes pentacycliques et les extraits de plantes (ex : Glycyrrhiza glabra) en contenant comme l'acide β-glycyrrhétinique et ses sels et/ou ses dérivés (l'acide glycyrrhétinique monoglucuronide, le stearyl glycyrrhetinate, l'acide 3- stéaroyloxy glycyrrhetique), l'acide ursolique et ses sels, l'acide oléanolique et ses sels, l'acide bétulinique et ses sels, un extrait de Paeonia suffruticosa et / ou lactiflora, les sels de l'acide salicylique et en particulier le salicylate de zinc, les phycosaccharides de la société Codif, un extrait de Laminaria saccharina, l'huile de Canola, le bisabolol et les extraits de camomille, l'allantoïne, le Sépivital EPC (diesterphosphorique de vitamine E et C) de Seppic, les huiles insaturées en oméga 3 telles que les huiles de rosier musca, de cassis, d'ecchium, de poisson, des extraits de plancton, la capryloyl glycine, le Seppicalm VG (sodium palmitoylproline et nymphea alba) de Seppic, un extrait du Pygeum, un extrait de Boswellia serrata, un extrait de Centipeda cunnighami, un extrait d'Helianthus annuus, un extrait de Linum usitatissimum, les tocotrienols, les extraits de Cola nitida, le piperonal, un extrait de clou de girofle, un extrait d'Epilobium Angustifolium, l'aloe vera, un extrait de Bacopa moniera, les phytostérols, la cortisone, l'hydrocortisone, l'indométhacine et la beta méthasone.

Les agents photoprotecteurs organiques sont notamment choisis parmi les filtres UVA , les filtres UVB et les filtres mixtes UVA-UVB et leurs mélanges.

### Filtres UVA

Le ou les filtres UVA selon la présente invention peu(ven)t être choisi(s) parmi les filtres suivants.

### 1) Filtres hydrophobes UVA

### Dérivés du dibenzoylméthane :

- Butyl Methoxydibenzoylméthane vendu notamment sous le nom commercial « PARSOL 1789 » par DSM Nutritional Products, Inc ;
- Isopropyl Dibenzoylméthane ;

### Aminobenzophénones :

- 2-(4-diéthylamino-2-hydroxybenzoyl)-benzoate de n-hexyle vendu notamment sous le nom commercial « UVINUL A +» par BASF ;

### Dérivés anthraniliques :

- Menthyl anthranilate vendu notamment sous le nom commercial « NEO HELIOPAN MA » par SYMRISE ;

### Dérivés de 4.4-diarylbutadiène :

- 1,1-dicarboxy (2,2'-diméthyl-propyl)-4,4-diphénylbutadiène ;

### Dérivés de mérocyanine :

- Octyl-5-N,N-diéthylamino-2-phénysulfonyl-2,4-pentadiénoate ;

### 2) Filtres hydrosolubles UVA

- Les dérivés du camphre tel que le Terephtalylidène Dicamphor Sulfonic Acid fabriqué sous le nom « MEXORYL SX » par CHIMEX,
- Les dérivés bis-benzoazolyle tels que décrits dans les brevets EP 669 323, et US 2,463,264 et plus particulièrement le composé Disodium Phenyl Dibenzimidazo tetra-sulfonate vendu sous le nom commercial « NEO HELIOPAN AP » par SYMRISE ;

Selon un mode de réalisation préféré ledit au moins un filtre UVA est
choisi parmi
les dérivés hydrophobes du dibenzoylméthane, les dérivés hydrosolubles du camphre et leurs mélanges.

Plus particulièrement, ledit au moins un filtre UVA est choisi parmi le butyl méthoxydibenzoyleméthane, le téréphtalylidène dicamphor sulfonic acid et leurs mélanges. De préférence, une composition selon l'invention est caractérisée en ce que ledit au moins un filtre UVA est présent dans une teneur allant de 1 à 20 %, en particulier, de 2 à 15 % en poids par rapport au poids total de la composition.

### Filtres UVB

Le ou les filtres UVB selon la présente invention peu(ven)t être choisi(s) parmi les filtres suivants.

### 1) Filtres hydrophobes UVB

### Para-aminobenzoates :

- Ethyl PABA ;
- Ethyl Dihydroxypropyl PABA ;
- Ethylhexyl Diméthyl PABA (ESCALOL 507 de ISP) ;

### Dérivés salicyliques :

- Homosalate vendu notamment sous le nom « Eusolex HMS » par Rona/EM Industries ;
- Ethylhexyl Salicylate vendu notamment sous le nom « NEO HELIOPAN OS » par SYMRISE ;
- Dipropylèneglycol Salicylate vendu notamment sous le nom « DIPSAL » par SCHER ;
- TEA Salicylate et sous le nom « NEO HELIOPAN TS » par SYMRISE ;

### Cinnamates

- Ethylhexyl Méthoxycinnamate vendu notamment sous le nom commercial « PARSOL MCX » par DSM Nutritional Products, Inc. ;
- Isopropyl Méthoxy cinnamate ;
- Isoamyl Méthoxy cinnamate vendu notamment sous le nom commercial « NEO HELIOPAN E 1000 » par SYMRISE ;
- Diisopropyl Méthylcinnamate ;
- Cinnoxate ;
- Glycéryl Ethylhexanoate Diméthoxycinnamate ;

### Dérivés de β,β'-diphénylacrylate :

- Octocrylène, vendu notamment sous le nom commercial « UVINUL N539 » par BASF ;
- Etocrylène, vendu notamment sous le nom commercial « UVINUL N35 » par BASF ;

### Dérivés du benzylidène camphre :

- 3-Benzylidène camphre fabriqué sous le nom « MEXORYL SD » par CHIMEX ;
- Méthylbenzylidène camphre vendu notamment sous le nom « EUSOLEX 6300 » par MERCK ;
- Polyacrylamidométhyle Benzylidène Camphre fabriqué sous le nom « MEXORYL SW » par CHIMEX ;

### Dérivés de triazine :

- Ethylhexyl triazone vendu notamment sous le nom commercial « UVINUL T150 » par BASF ;
- Diéthylhexyl Butamido Triazone vendu notamment sous le nom commercial « UVASORB HEB » par SIGMA 3V ;
- 2,4,6-tris(4'-amino benzalmalonate de dinéopentyle)-s-triazine ;
- 2,4,6-tris(4'-amino benzalmalonate de diisobutyle)-s- triazine ;
- 2,4-bis(4'-amino benzalmalonate de dinéopentyle)-6-(4'-aminobenzoate de n-butyle)-s-triazine ;
- 2,4-bis(4'-amino benzoate de n-butyle)-6-(aminopropyltrisiloxane)-s-triazine ;
- les filtres triazines symétriques décrits dans le brevet US 6,225,467, la demande WO 2004/085412 (voir composés 6 et 9) ou le document « Symetrical Triazine Derivatives » IP.COM Journal, IP.COM INC WEST HENRIETTA, NY, US (20 septembre 2004) notamment les 2,4,6-tris-(biphényl)-1,3,5-triazine (en particulier la 2,4,6-tris(biphényl-4-yl-1,3,5-triazine) et la 2,4,6-tris(terphenyl)-1,3,5-triazine, ces deux derniers filtres étant décrits dans les demandes de BEIERSDORF WO 06/035000, WO 06/034982, WO 06/034991, WO 06/035007, WO 2006/034992, WO 2006/034985).

### Dérivés d'imidazolines :

- Ethylhexyl Diméthoxybenzylidene Dioxoimidazoline Propionate,

### Dérivés du benzalmalonate :

- Polyorganosiloxanes à fonction benzalmalonate tels que le Polysilicone-15 vendu notamment sous la dénomination commerciale « PARSOL SLX » par DSM Nutritional Products, Inc. ;
- Di-neopentyl 4'-méthoxybenzalmalonate ;

### 2) Filtres hydrosolubles UVB

Les dérivés d'acide p-aminobenzoïque (PABA) suivants :
- PABA,
- Glyceryl PABA et
- PEG-25 PABA vendu notamment sous la dénomination commerciale « UVINUL P25 » par BASF.
- le Phenylbenzimidzaole Sulfonic Acid vendu notamment sous la dénomination commerciale « EUSOLEX 232 » par MERCK,
- l'acide férulique,
- l'acide salicylique,
- le DEA methoxycinnamate,
- le benzylidène camphre Acide Sulfonique fabriqué sous le nom « MEXORYL SL » par CHIMEX,
- le camphre Benzalkonium Methosulfate fabriqué sous le nom « MEXORYL SO » par CHIMEX.

Selon un mode de réalisation préféré, le au moins un filtre UVB compris dans une composition selon l'invention est un filtre hydrophobe UVB.

En particulier, ledit au moins un filtre hydrophobe UVB est choisi parmi les dérivés salicyliques, les cinnamates, les dérivés de □,□'-diphénylacrylate, les dérivés de triazine et leurs mélanges.

De préférence, le moins un filtre UVB est choisi parmi l'éthylhexyl salicylate, l'éthylhexyl méthoxycinnamate, l'octocrylène, l'éthylhexyl triazone et leurs mélanges.

De préférence, une composition selon l'invention est caractérisée en ce que ledit au moins un filtre UVB est présent dans une teneur allant de 1 à 20 %, en particulier de de 5 à 15 %, et de préférence de 7 à 10 % en poids par rapport au poids total de la composition.

Selon une variante particulière de l'invention, ledit système filtrant organique utilisé dans l'invention contient voire est constitué d'un ou plusieurs filtre(s) mixte(s) UVA-UVB.

Avantageusement, ledit système filtrant organique utilisé dans l'invention contient un ou plusieurs filtres UVA, un ou plusieurs filtres UVB et un ou plusieurs filtres mixte UVA-UVB.

### Filtres mixtes UVA et UVB

Ledit au moins un filtre mixte UVA-UVB peut être choisi parmi les filtres suivants.

### 1) Filtres hydrophobes mixtes UVA et UVB

### Dérivés de benzophénone

- Benzophénone-1 vendu notamment sous le nom commercial « UVINUL 400 » par BASF ;
- Benzophénone-2 vendu notamment sous le nom commercial « UVINUL D50 » par BASF ;
- Benzophénone-3 ou Oxybenzone vendu notamment sous le nom commercial « UVINUL M40 » par BASF ;
- Benzophénone-6 vendu notamment sous le nom commercial « Helisorb 11 » par Norquay ;
- Benzophénone-8 vendu notamment sous le nom commercial « Spectra-Sorb UV-24 » par American Cyanamid ;
- Benzophénone-10 ;
- Benzophénone-11 ;
- Benzophénone-12 ;

### Dérivés du phényl benzotriazole :

- Drométrizole Trisiloxane vendu notamment sous le nom « Silatrizole » par RHODIA CHIMIE ou fabriqué sous le nom « Meroxyl XL » par la société CHIMEX ;
- Méthylène bis-Benzotriazolyl Tétramethylbutylphénol, vendu sous forme solide notamment sous le nom commercial « MIXXIM BB/100 » par FAIRMOUNT CHEMICAL ou sous forme micronisée en dispersion aqueuse notamment sous le nom commercial « TINOSORB M » par CIBA SPECIALTY CHEMICALS ;

### Dérivés bis-résorcinyl triazines

- Bis-Ethylhexyloxyphénol Méthoxyphenyl Triazine vendu notamment sous le nom commercial « TINOSORB S » par CIBA GEIGY ;

### Dérivés de benzoxazole :

- 2,4-bis-[5-1(diméthylpropyl)benzoxazol-2-yl-(4-phenyl)-imino]-6-(2-ethylhexyl)-imino-1,3,5-triazine vendu notamment sous le nom d'Uvasorb K2A par Sigma 3V.

### 2) Filtres hydrosolubles mixtes UVA et UVB

Dérivés de benzophénone comportant au moins un radical sulfonique comme
- Benzophénone-4 vendu notamment sous le nom commercial « UVINUL MS 40 » par BASF,
- Benzophénone-5, et
- Benzophénone-9.

Les agents photoprotecteurs inorganiques sont choisis parmi des pigments (de tailles variables) d'oxydes métalliques enrobés ou non comme par exemple des pigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels pigments d'oxydes métalliques, enrobés ou non enrobés, sont en particulier décrits dans les demandes de brevets EP518772 et EP518773.

Les agents photoprotecteurs lorsque présents, représentent de 0,1 à 20 % en poids par rapport au poids total de la composition, et de préférence de 0,2 à 15 % en poids par rapport au poids total de la composition.

Les exemples qui suivent illustrent l'invention sans en limiter la portée. Les composés sont, selon le cas, cités en noms chimiques ou en noms CTFA (International Cosmetic Ingredient Dictionary and Handbook).

### Exemple 1 : Synthèse du composé 11

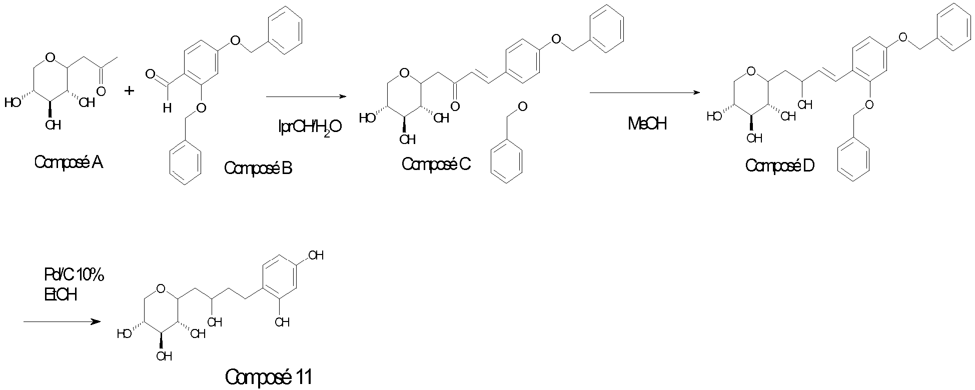

### Synthèse du composé C :

Le composé A (418 mg) est mis en agitation dans un mélange IprOH/H₂O (40/10 ml) en présence de 3 équivalents d'hydroxyde de lithium. Le milieu est chauffé à 50°C pendant 30 min puis le composé B (4.9 g) est additionné. La température est maintenue pendant 2h. Après retour à température ambiante, le milieu réactionnel est concentré sous vide puis purifié par chromatographie sur colonne de silice (dichlorométhane/méthanol 95/5). Le composé intermédiaire C est obtenu en mélange avec des impuretés non caractérisées.

### Synthèse du composé D

1.1 g du composé C tel qu'obtenu dans l'étape précédente est solubilisé dans 15 ml de MeOH. Le borohydrure de sodium (2 éq) est ajouté progressivement. Après fin du dégagement gazeux, on ajoute 20 ml d' acétone. Le milieu réactionnel est ensuite passé sur résine DOWEX 50WX8 puis Amberlite IRA 743. Les filtrats sont concentrés sous vide et le résidu brut obtenu est mis en solution dans de l'acétate d'éthyle puis lavé 2 fois avec une solution saturée de NaCl. La phase organique est recueillie puis concentrée sous vide. On obtient ainsi le composé D sous forme de poudre jaune. Le composé D sera engagé sans purification supplémentaire dans l'étape suivante.

### Synthèse du composé 11:

Le composé D est solubilisé dans un mélange Cyclohexène/EtOH 50/50 8ml/8ml puis 235 mg de Pd/C 10% sont ajoutés. Le mélange est porté à reflux pendant 2h. Le milieu réactionnel est filtré sur un lit de Célite puis purifié sur colonne chromatographique de silice (Dichlorométhane/méthanol 80/20). Le composé 11 est obtenu sous forme d'une poudre beige avec un rendement de 29%. Les spectres RMN et spectres de Masse sont conformes à la structure **11**.

### Exemple 2 : Synthèse du composé 10

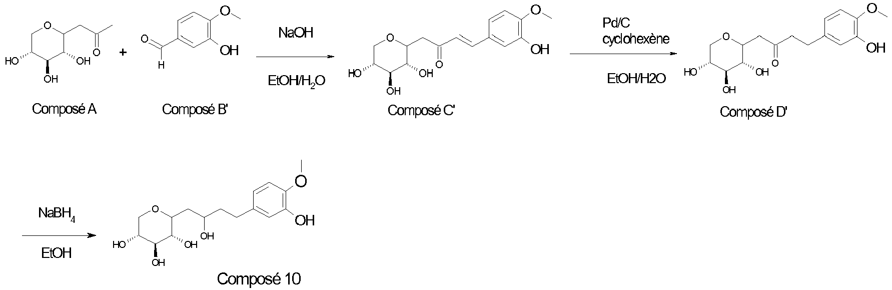

### Synthèse du composé C' :

Le composé A (4g) est mis en agitation dans un mélange EtOH/H₂O (40/10 ml) en présence de 3.5 équivalents d'hydroxyde de sodium dans 27 ml d'eau. Le milieu est chauffé à 50°C pendant 30 min puis le composé B' (3.84 g) est additionné. La température est maintenue pendant 2h. Après retour à température ambiante, le milieu réactionnel est concentré sous vide puis purifié par chromatographie sur colonne de silice (dichlorométhane/méthanol 95/5). Le composé intermédiaire C' est obtenu en mélange avec des impuretés non caractérisées avec un rendement de 38% (2.6g).

### Synthèse du composé D'

1.87 g du composé C' tel qu'obtenu dans l'étape précédente est solubilisé dans 50ml d'éthanol avec 17 ml d'eau et 13ml de cyclohexène. 500 mg de Pd/C sont ajoutés et le milieu réactionnel est chauffé au reflux pendant 18h. Après refroidissement, le mélange est filtré sur célite et rincé à l'éthanol. Après concentration le brut sous forme d'un solide gris est engagé dans l'étape suivante sans purification supplémentaire.

### Synthèse du composé 10:

Le composé D' tel qu'obtenu dans l'étape précédente est solubilisé dans 120 ml d'éthanol et 20 ml de méthanol. Le borohydrure de sodium (2 éq 460 mg) est ajouté progressivement. Après fin du dégagement gazeux, on ajoute 20 ml d' acétone puis une solution aqueuse saturée de chlorure d'ammonium. Le brut obtenu après concentration est ensuite purifié par chromatographie sur colonne de silice avec pour éluant un mélange dichlorométhane/méthanol 80/20 pour conduire à 1.58 g d'un miel beige identifié comme le composé 10 sous forme d'un mélange de diastéréosiomères.

### Exemple 3 : Mise en évidence de l'activité sur la mélanogénèse.

Un test biologique a mis en évidence l'activité dépigmentante du composé 11 de l'exemple 1.

L'effet modulateur sur la mélanogénèse du composé de l'exemple 1 a été mesuré selon la méthode décrite dans le brevet FR-A-2734825 , ainsi que dans l'article de R.Schmidt, P. Krien et M. Régnier, Anal. Bichem., 235(2), 113-18,1996. Ce test est réalisé sur coculture de kératinocytes et de mélanocytes.

Pour le composé testé, il a été déterminé :
- la cytotoxicité, en estimant l'incorporation de la leucine,
- l'activité inhibitrice sur la synthèse de la mélanine, en estimant le rapport de l'incorporation de thiouracile à l'incorporation de leucine, rapporté à 100 % du témoin (le témoin correspond au test réalisé sans composé à testé). Les valeurs des IC50 (concentration pour laquelle 50 % de la synthèse de la mélanine est inhibée) ont été déterminées.

On a également effectué le test avec l'arbutine et l'acide kojique qui sont des composés dépigmentant connus.

Les résultats sont rassemblés dans le tableau suivant :

| **Composé** | **Cytotoxicité sur co-culture** | **IC50** |
|---|---|---|
| Arbutine | Non cytotoxique | Non atteinte (ou supérieure à 500 µM) |
| Acide kojique | 100 µM | Non atteinte (ou supérieure à 500 µM) |
| Composé 11 | Non cytotoxique | 145 µM |
| 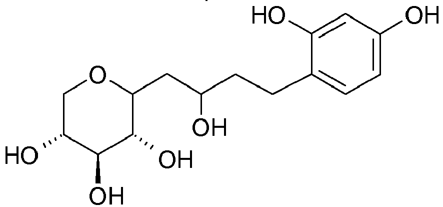 | | |
| Exemple 1 | | |

Le composé 11 présente une activité dépigmentante supérieure à celle de l'acide kojique et à l'arbutine.

### Mise en évidence de l'activité du composé 1 sur la pigmentation dans un modèle de peau reconstruite complet comprenant un derme vivant.

L'activité du composé **1** a été évaluée sur le modèle de peau reconstruite complet décrit dans le brevet FR2928654 selon le procédé décrit dans le brevet FR2930644.

Le composé 1 a été ajouté au milieu de culture pendant une période de 16 jours à partir du 2eme jour de la phase d'émersion. La pigmentation a été induite par la Forskoline ajoutée pendant une période de 14 jours à partir du 4eme jour d'émersion (condition contrôle positif pigmenté).

L'absence d'effet toxique de la molécule a été vérifié par histologie (morphologie de la peau reconstruite) et par quantification du nombre de mélanocytes (marquage TRP1 et réaction DOPA sur feuillet épidermique).

Le composé **1** a montré une activité dépigmentante sur les paramètres suivants :
**Luminance (clarté de la peau)** plus la valeur de luminance est élevée plus l'échantillon de peau est clair :
   augmentation significative de 6 points de la luminance pour le composé**1** (Valeur de L contrôle positif = 63, valeur de L traitement par molécule **1** à 1 mM = 69)
**Quantification de la mélanine par analyse d'images sur des coupes colorées au Fontana Masson (coloration argentique des grains de mélanine) :** réduction de 60% de la quantité de mélanine par le composé 1 (contrôle positif = valeur arbitraire de 100%, composé 1 à 1 mM = 39,48%).

### Exemple 4 : Synthèse du composé 13

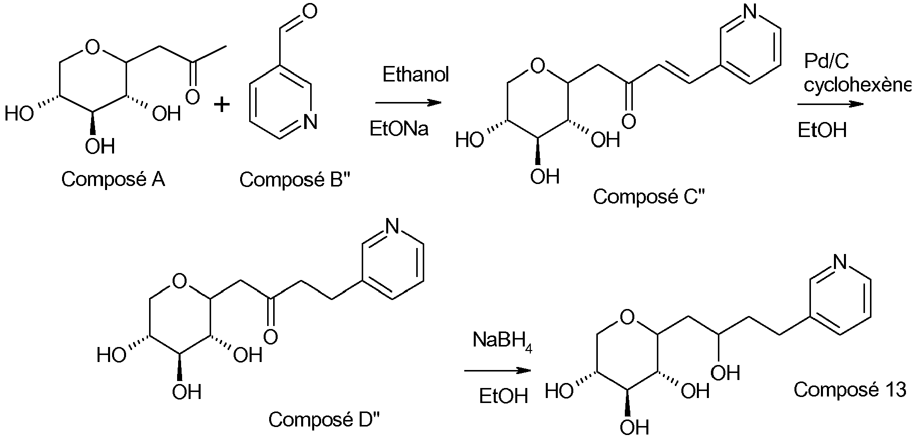

### Synthèse du composé C" :

Le composé A (1g) a été mis en agitation dans 30 ml d'éthanol. Le milieu a été chauffé à 60°C pendant 30 min puis le composé B" (0,54ml) a été additionné. On aadditionné ensuite lentement 1.8ml d'éthylate de sodium à 20% dans l'éthanol (5.21 mmoles). On a laissé réagir 1 h à 60°C puis 7h jusqu'au retour à température ambiante.

Après avoir vérifié la consommation totale du composé A, le milieu réactionnel a été concentré sous vide puis purifié par chromatographie sur colonne de silice (dichlorométhane/méthanol 95/5). Le composé intermédiaire C" a été obtenu en mélange avec des impuretés non caractérisées avec un rendement de 28% (2.6g).La caractérisation par RMN était conforme au produit attendu.

### Synthèse du composé D"

0,4 g du composé C" tel qu'obtenu dans l'étape précédente a été solubilisé dans 20ml d'éthanol et 5ml de cyclohexène. 500 mg de Pd/C ont été ajoutés et le milieu réactionnel a été chauffé au reflux pendant 4h.

Après avoir vérifié la disparition totale du produit de départ, le milieu a été filtré sur papier filtre pour éliminer le catalyseur puis concentré au rotavapor.

On a récupéré 0.2g du composé D" sous forme d'une pate incolore avec un rendement de 50%. La caractérisation par RMN était conforme au produit attendu.

### Synthèse du composé 13:

Dans un ballon, on a solubilisé 0.2g du composé D" (0.71 mmoles) dans 10ml d'éthanol. On a ajouté 0.2g de borohydrure de sodium en granules (5.3 mmoles) et on a laissé réagir à température ambiante pendant 3h.

Après avoir vérifié la disparition totale du produit de départ, le milieu a été concentré au rotavapor puis purifié sur silice.

On a récupéré 50mg du composé 13 sous forme d'un solide beige avec un rendement de 25%. La caractérisation par RMN était conforme au produit attendu.

### Exemples de compositions

### Exemple A :

Crème Eau dans Huile (E/H):

| | |
|---|---|
| Octyl dodécanol | 1% |
| Stéarate de magnésium | 4% |
| Cire d'abeille naturelle | 5% |
| Sesquioléate de sorbitan | 4,5% |
| Mono et distéarate de glycérol et stéarate de potassium | 1,0% |
| Huile de vaseline | 22% |
| Huile de Jojoba | 4% |
| Composé 1 | 2,5% |
| Conservateurs | 0,4% |
| Parfum | 0,6% |
| Eau | qsp 100% |

### Exemple B :

On prépare une crème blanchissante de soin du visage de type émulsion huile dans eau, comprenant (% en poids) :

| | |
|---|---|
| Composé 1 | 3% |
| Stéarate de glycérol | 2% |
| Polysorbate 60 (Tween 60 de ICI) | 1% |
| Acide stéarique | 1,4% |
| Triéthanolamine | 0,7% |
| Carbomer | 0,4% |
| Fraction liquide du beurre de karité | 12% |
| Perhydrosqualène | 12% |
| Antioxydant | 0,05% |
| Parfum, conservateur | qs |
| Eau | qsp 100% |

### Exemple C :

On prépare un gel dépigmentant pour la peau comprenant (% en poids) :

| | |
|---|---|
| Composé 11 (exemple 1) | 2% |
| hydroxypropylcellulose (Klucel H de Hercules) | 1% |
| antioxydant | 0,05% |
| isopropanol | 40% |
| parfum, conservateur | qs |
| eau | qsp 100% |

## Revendications

1. Procédé cosmétique de dépigmentation, d'éclaircissement et/ou de blanchiment de la peau, des poils ou des cheveux comprenant l'application sur la peau, les poils ou les cheveux d'une composition comprenant, dans un milieu physiologiquement acceptable, au moins un composé de formule (I) : dans laquelle, 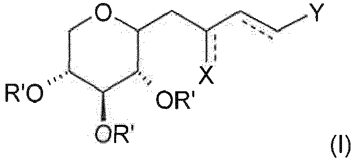
- les composés de formule (I) sont des dérivés de xylose
- Y désigne un radical phényle ou un hétérocycle, éventuellement substitués par 1 à 5 groupements (ORₐ)
- X = -OR' ; (=O) ; NR_{b}R_{c} ; NHOR_{d}
- R' désigne :
* un atome d'hydrogène,
* un radical alkyle linéaire et saturé en C1-C18,
*un radical alkyle linéaire et insaturé C2-C18
* un radical alkyle ramifié, saturé ou insaturé, en C3-C18
* un radical cyclique saturé ou insaturé en C5 ou C6
* un radical acyle, linéaire ou ramifié, saturé ou insaturé, en C2-C18, ou cyclique saturé ou insaturé en C5 ou C6.
**Rₐ** désigne :
- un atome d'hydrogène
- un radical alkyle linéaire ou ramifié, en C1-C4, ou un radical hydrocarboné insaturé linéaire ou ramifié en C3-C4
- un radical acyle linéaire ou ramifié en C2-C18 ou alcénylcarbonyle linéaire ou ramifié en C2-C18
Lorsque Y désigne un radical phényle ou un hétérocycle substitué par 2 à 5 groupements (ORₐ), deux groupements adjacents ORₐ peuvent former ensemble un radical divalent -O-CH₂-O
sous réserve que lorsque X=OH, le composé ne comporte pas de double liaison éthylénique en position alpha du carbone portant cet OH.
**R_{b}** désigne :
- un atome d'hydrogène
- un radical alkyle linéaire en C2-C18, ou ramifié en C3-C18, ou un radical hydrocarboné insaturé linéaire ou ramifié en C3-C4 ou un radical -CH(Z₁)-CO₂Z₂ dans lequel
Z₁ désigne un atome d'hydrogène ou un radical alkyle linéaire ou ramifié en C1-C6, ou cyclique saturé ou insaturé en C3-C6, ledit radical étant éventuellement substitué par au moins un groupement choisi parmi =NH, -NH₂, -N(T)₂, =O, -OH, - OT, -SH, -ST, -CO₂T, phényle, phényle substitué par -OH ou -OT, 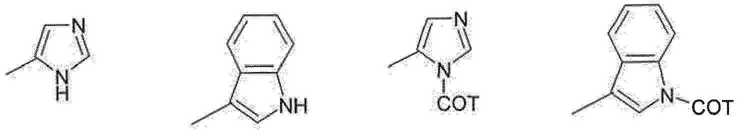 et/ou interrompu par un groupement-NH-, -N-(COT)-, ou -S- avec T désignant un radical alkyle linéaire ou ramifié en C1-C6, ou cyclique en C3-C6.
et Z₂ désigne un atome d'hydrogène ou un radical alkyle linéaire ou ramifié en C1-C6
**R_{c}** désigne :
- un atome d'hydrogène
- un radical alkyle linéaire en C1-C4, ou ramifié en C3-C4, ou un radical hydrocarboné insaturé linéaire ou ramifié en C3-C4, ledit radical étant éventuellement substitué par un groupement phényle.
**Rd** désigne :
- un atome d'hydrogène
- un radical alkyle linéaire en C1-C18, ou ramifié en C3-C18, ou un radical hydrocarboné insaturé linéaire ou ramifié en C3-C18, ledit radical étant éventuellement substitué par un groupe phényle,
ainsi que leurs sels cosmétiquement acceptables, leurs solvates tels que les hydrates et leurs stéréoisomères,

2. Procédé selon la revendication précédente, **caractérisé par** la fait que :
- les composés de formule (I) sont des dérivés de xylose
- Y désigne un radical phényle ou un hétérocycle, éventuellement substitués par 1 à 5 groupements (ORₐ)
- X = -OR' ; (=O) ; NR_{b}R_{c} ; NHOR_{d}
- R' désigne :
* un atome d'hydrogène,
* un radical alkyle linéaire ou ramifié, saturé ou insaturé en C1-C12, ou cyclique saturé en C5 ou C6
* un radical acyle linéaire ou ramifié, saturé ou insaturé en C2-C6, ou cyclique saturé en C5 ou C6.
**Rₐ** désigne :
- un atome d'hydrogène
- un radical alkyle linéaire ou ramifié, en C1-C4,
- un radical acyle linéaire ou ramifié en C2-C6
Lorsque Y désigne un radical phényle ou un hétérocycle substitué par 2 à 5 groupements (ORₐ), deux groupements adjacents ORₐ peuvent former ensemble un radical divalent -O-CH₂-O
sous réserve que lorsque X=OH, le composé ne comporte pas de double liaison éthylénique en position alpha du carbone portant cet OH.
**R_{b}** désigne :
- un atome d'hydrogène
- un radical alkyle linéaire en C2-C12, ou ramifié en C3-C12, ou un radical-CH(Z₁)-CO₂Z₂ dans lequel
Z₁ désigne un atome d'hydrogène ou un radical alkyle linéaire ou ramifié en C1-C6, ou cyclique saturé ou insaturé en C3-C6, ledit radical étant éventuellement substitué par au moins un groupement choisi parmi =NH, -NH₂, -N(T)₂, =O, -OH,-OT, -SH, -ST, -CO₂T, phényle, phényle substitué par -OH ou -OT, 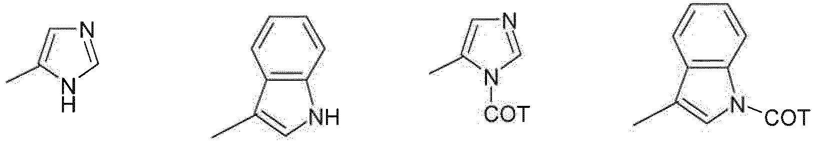 et/ou interrompu par un groupement -NH-, -N-(COT)-, ou -S- avec T désignant un radical alkyle linéaire en C1-C6, cyclique en C5-C6.
et Z₂ désigne un atome d'hydrogène ou un radical alkyle linéaire ou ramifié en C1-C6
**R_{c}** désigne :
- un atome d'hydrogène
- un radical alkyle linéaire en C1-C4, ou ramifié en C3-C4, ou un radical hydrocarboné insaturé linéaire ou ramifié en C3-C4, ledit radical étant éventuellement substitué par un groupement phényle.
**Rd** désigne :
- un atome d'hydrogène
- un radical alkyle linéaire en C1-C12, ou ramifié en C3-C12, ledit radical étant éventuellement substitué par un groupe phényle,
ainsi que leurs sels cosmétiquement acceptables, leurs solvates tels que les hydrates et leurs stéréoisomères,

3. Procédé selon l'une quelconque des revendications précédentes, **caractérisé par** la fait que :
- les composés de formule (I) sont des dérivés de xylose
- Y désigne un radical phényle ou un hétérocycle, éventuellement substitués par 1 à 3 groupements (ORₐ)
- X = -OR' ; (=O) ; NR_{b}R_{c} ; NHOR_{d}
- R' désigne :
* un atome d'hydrogène,
* un radical alkyle linéaire ou ramifié, saturé ou insaturé en C1-C4,
* un radical acyle linéaire ou ramifié en C2-C6.
**Rₐ** désigne:
- un atome d'hydrogène
- un radical alkyle linéaire ou ramifié, en C1-C4,
- un radical acyle linéaire ou ramifié en C2-C6
Lorsque Y désigne un radical phényle ou un hétérocycle substitué par 2 ou 3 groupements (ORₐ), deux groupements adjacents ORₐ peuvent former ensemble un radical divalent -O-CH₂-O
sous réserve que lorsque X=OH, le composé ne comporte pas de double liaison éthylénique en position alpha du carbone portant cet OH.
**R_{b}** désigne :
- un atome d'hydrogène
- un radical alkyle linéaire en C2-C8, ou ramifié en C3-C8, ou un radical-CH(Z₁)-CO₂Z₂ dans lequel
Z₁ désigne un atome d'hydrogène ou un radical alkyle linéaire ou ramifié en C1-C6, ou cyclique saturé ou insaturé en C3-C6, ledit radical étant éventuellement substitué par au moins un groupement choisi parmi =NH, -NH₂, -N(T)₂, =O, -OH,-OT, -SH, -ST, -CO₂T, phényle, phényle substitué par -OH ou -OT, 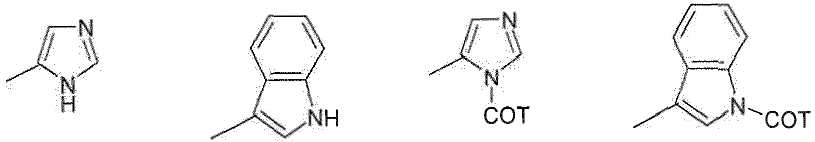 et/ou interrompu par un groupement -NH-, -N-(COT)-, ou -S- avec T désignant un radical alkyle linéaire en C1-C6, cyclique en C5-C6.
et Z₂ désigne un atome d'hydrogène ou un radical alkyle linéaire ou ramifié en C1-C6
**R_{c}** désigne :
- un atome d'hydrogène
- un radical alkyle linéaire en C1-C4, ou ramifié en C3-C4, éventuellement substitué par un groupement phényle.
**Rd** désigne :
- un atome d'hydrogène
- un radical alkyle linéaire en C1-C4, ou ramifié en C3-C4, ledit radical étant éventuellement substitué par un groupe phényle,
ainsi que leurs sels cosmétiquement acceptables, leurs solvates tels que les hydrates et leurs stéréoisomères,

4. Procédé selon l'une quelconque des revendications précédentes, **caractérisé par** la fait que le composé de formule (I) est choisi parmi :
**Composé 1. :** (3R,4S,5R)-2-[2-hydroxy-4-(4-hydroxy-3-methoxyphenyl)butyl]tetrahydro-2H-pyran-3,4,5-triol 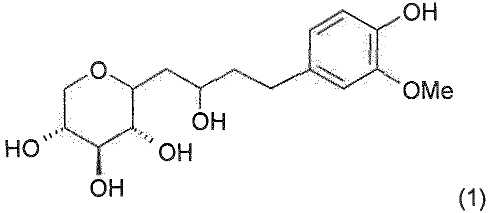
**Composé 2.** 4-(4-hydroxy-3-methoxyphenyl)-1-[(3R,4S,5R)-3,4,5-trihydroxytetrahydro-2H-pyran-2-yl]butan-2-one 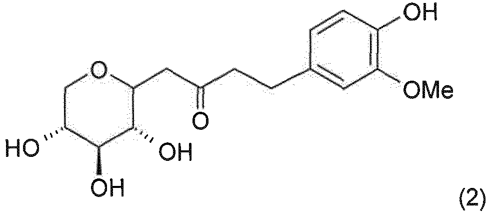
**Composé 3.** (3R,4S,5R)-2-[2-hydroxy-4-(4-hydroxy-phenyl)butyl]tetrahydro-2H-pyran-3,4,5-triol 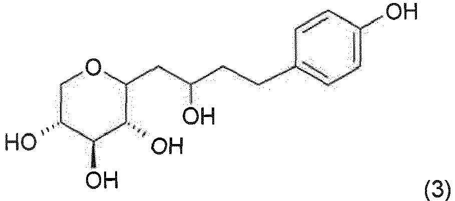
**Composé 4.** 4-(4-hydroxy-phenyl)-1-[(3R,4S,5R)-3,4,5-trihydroxytetrahydro-2H-pyran-2-yl]butan-2-one 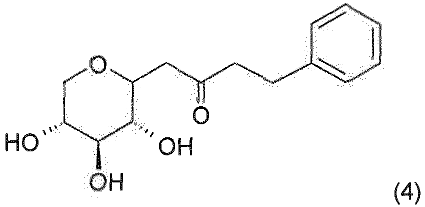
**Composé 5.** (3R,4S,5R)-2-[2-(benzylamino)-4-(4-hydroxy-3-methoxyphenyl)butyl]tetrahydro-2H-pyran-3,4,5-triol 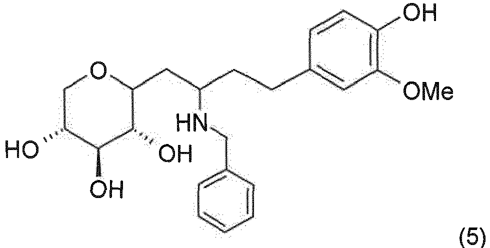
**Composé 6.** ethyl {[3-(4-hydroxy-3-methoxyphenyl)-1-{[(3R,4S,5R)-3,4,5-trihydroxytetrahydro-2H-pyran-2-yl]methyl}propyl]amino}(phenyl)acetate 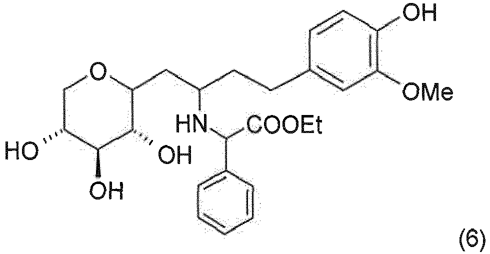
**Composé 7.** (3E)-4-phenyl-1-[(3R,4S,5R)-3,4,5-trihydroxytetrahydro-2H-pyran-2-yl]but-3-en-2-one 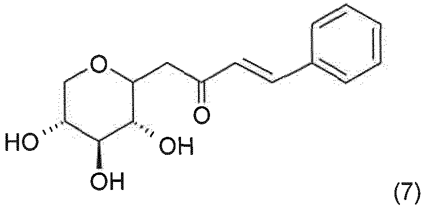
**Composé 8.** (3E)-4-(4-hydroxy-3,5-dimethoxyphenyl)-1-[(3R,4S,5R)-3,4,5-trihyd roxytetrahydro-2H-pyran-2-yl]but-3-en-2-one 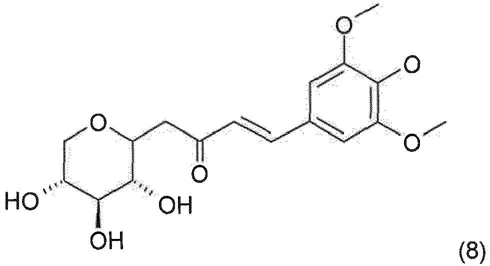 <
**Composé 9.** (3R,4S,5R)-2-[2-hydroxy-4-(2-hydroxyphenyl)butyl]tetrahydro-2H-pyran-3,4,5-triol 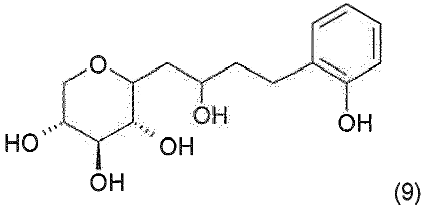
**Composé 10 :** (3R,4S,5R)-2-[2-hydroxy-4-(3-hydroxy-4-methoxyphenyl)butyl]tetrahydro-2H-pyran-3,4,5-triol 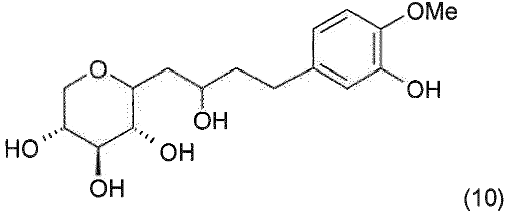
**Composé 11.** (3R,4S,5R)-2-[2-hydroxy-4-(2,4-di-hydroxy-phenyl)butyl]tetrahydro-2H-pyran-3,4,5-triol 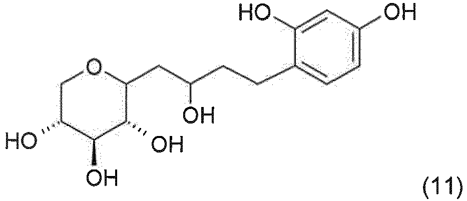
**Composé 12.** (3R,4S,5R)-2-[2-hydroxy-4-(3-ethoxy-4-hydroxy-phenyl)butyl]tetrahydro-2H-pyran-3,4,5-triol 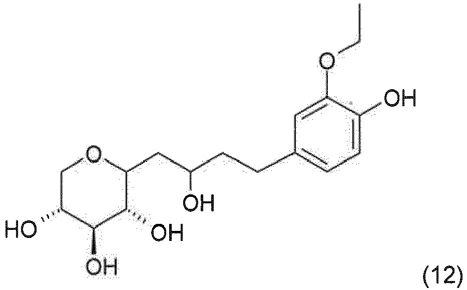
**Composé 13.** 5,9-anhydro-1,2,4-trideoxy-1-pyridin-3-yl-D-xylo-nonitol 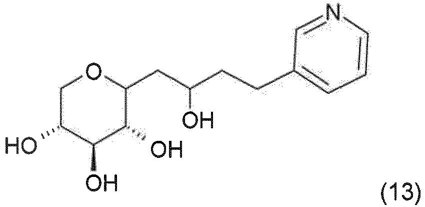
**Composé 14.** (3R,4S,5R)-2-[(2E)-2-(methoxyimino)-4-phenylbutyl]tetrahydro-2H-pyran-3,4,5-triol 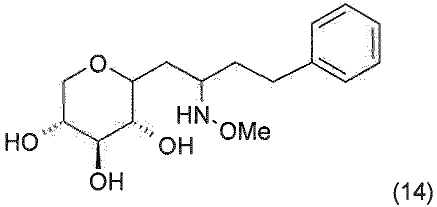
**Composé 15.** 5,9-anhydro-1,2,4-trideoxy-7-O-pentanoyl-1-phenyl-D-xylo-non-3-ulose 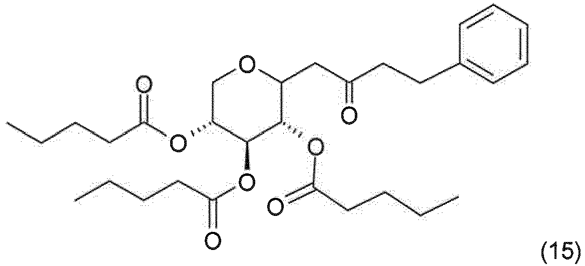
**Composé 16.** 5,9-anhydro-1,2,4-trideoxy-1-(3,4,5-trimethoxyphenyl)-D-xylononitol

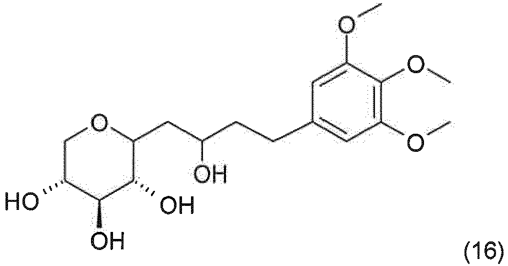

5. Procédé selon l'une quelconque des revendications précédentes, **caractérisé par** la fait que le composé de formule (I) est choisi parmi :
**Composé 11.** (3R,4S,5R)-2-[2-hydroxy-4-(2,4-di-hydroxy-phenyl)butyl]tetrahydro-2H-pyran-3,4,5-triol 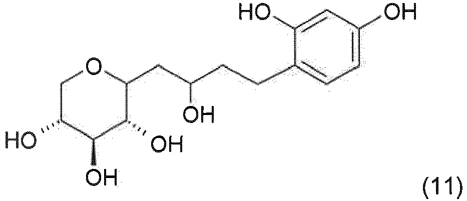
**Composé 12.** (3R,4S,5R)-2-[2-hydroxy-4-(3-ethoxy-4-hydroxy-phenyl)butyl]tetrahydro-2H-pyran-3,4,5-triol

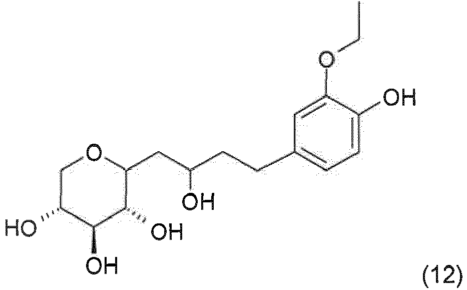

6. Procédé selon l'une quelconque des revendications précédentes, **caractérisé par** la fait que le composé de formule (I) est présent en une quantité allant de 0,001% à 10% en poids, par rapport au poids total de la composition, de préférence allant de 0,01% à 5% en poids, notamment de 0,1 à 2%.

7. Utilisation cosmétique d'un composé de formule (I) tel que défini selon l'une quelconque des revendications 1 à 6, comme agent blanchissant, éclaircississant et/ou dépigmentant de la peau, des poils ou des cheveux.

8. Composés C-xylosides de formule (II) : 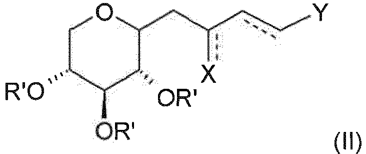 dans laquelle,
- les composés de formule (II) sont des dérivés de xylose
- Y désigne un hétérocycle, éventuellement substitué par 1 à 5 groupements (ORₐ)
- X = -OR' ; (=O) ; NR_{b}R_{c} ; NHOR_{d}
**R'** désigne :
* un atome d'hydrogène,
* un radical alkyle linéaire et saturé en C1-C18,
*un radical alkyle linéaire et insaturé C2-C18
* un radical alkyle ramifié, saturé ou insaturé, en C3-C18
* un radical cyclique saturé ou insaturé en C5 ou C6
* un radical acyle, linéaire ou ramifié, saturé ou insaturé, en C2-C18, ou cyclique saturé ou insaturé en C5 ou C6.
**Rₐ** désigne :
- un atome d'hydrogène
- un radical alkyle linéaire ou ramifié, en C1-C4, ou un radical hydrocarboné insaturé linéaire ou ramifié en C3-C4
- un radical acyle linéaire ou ramifié en C2-C18 ou alcénylcarbonyle linéaire ou ramifié en C2-C18,
**R_{b}** désigne :
- un atome d'hydrogène
- un radical alkyle linéaire en C2-C18, ou ramifié en C3-C18, ou un radical hydrocarboné insaturé linéaire ou ramifié en C3-C4 ou un radical -CH(Z₁₎-CO₂Z₂ dans lequel
Z₁ désigne un atome d'hydrogène ou un radical alkyle linéaire ou ramifié en C1-C6, ou cyclique saturé ou insaturé en C3-C6, ledit radical étant éventuellement substitué par au moins un groupement choisi parmi =NH, -NH₂, -N(T)₂, =O, -OH, - OT, -SH, -ST, -CO₂T, phényle, phényle substitué par -OH ou -OT, 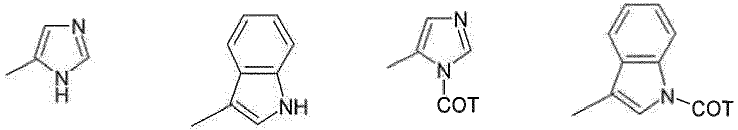 et/ou interrompu par un groupement -NH-, -N-(COT)-, ou -S- avec T désignant un radical alkyle linéaire ou ramifié en C1-C6, ou cyclique en C3-C6.
et Z₂ désigne un atome d'hydrogène ou un radical alkyle linéaire ou ramifié en C1-C6
**Rc** désigne :
- un atome d'hydrogène
- un radical alkyle linéaire en C1-C4, ou ramifié en C3-C4, ou un radical hydrocarboné insaturé linéaire ou ramifié en C3-C4, ledit radical étant éventuellement substitué par un groupement phényle.
**Rd** désigne:
- un atome d'hydrogène
- un radical alkyle linéaire en C1-C18, ou ramifié en C3-C18, ou un radical hydrocarboné insaturé linéaire ou ramifié en C3-C18, ledit radical étant éventuellement substitué par un groupe phényle,
ainsi que leurs sels cosmétiquement acceptables, leurs solvates tels que les hydrates et leurs stéréoisomères,

9. Composés C-xylosides de formule (III) : dans laquelle, 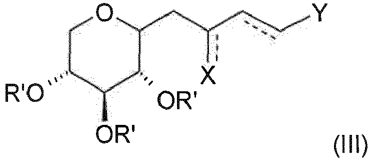
- les composés de formule (III) sont des dérivés de xylose
- **Y** désigne un radical phényle éventuellement substitué par 1 à 5 groupements (ORₐ)
- **X** = -OR" ; NR_{b}R_{c} ; NHOR_{d}
- **R'** désigne :
* un atome d'hydrogène,
* un radical alkyle linéaire ou ramifié, saturé ou insaturé en C1-C18, , ou cyclique saturé ou insaturé en C5 ou C6
* un radical acyle linéaire ou ramifié, saturé ou insaturé en C1-C18, ou cyclique saturé ou insaturé en C5 ou C6.
- **R"** désigne :
* un radical alkyle linéaire ou ramifié, saturé ou insaturé en C1-C18, , ou cyclique saturé ou insaturé en C5 ou C6
* un radical acyle linéaire ou ramifié, saturé ou insaturé en C1-C18, , ou cyclique saturé ou insaturé en C5 ou C6.
**Rₐ** désigne :
- un atome d'hydrogène
- un radical alkyle linéaire ou ramifié en C1-C4, ou un radical hydrocarboné insaturé linéaire ou ramifié en C3-C4
- un radical acyle linéaire ou ramifié en C1-C18 ou alcénylcarbonyle linéaire ou ramifié en C2-C18
Lorsque **Y** désigne un radical phényle substitué par 2 à 5 groupements (ORₐ), deux groupements adjacents ORₐ peuvent former ensemble un radical divalent-O-CH₂-O,
**R_{b}** désigne :
- un atome d'hydrogène
- un radical alkyle linéaire en C2-C18, ou ramifié en C3-C18, ou un radical hydrocarboné insaturé linéaire ou ramifié en C3-C4 ou un radical -CH(Z₁)-CO₂Z₂ dans lequel
Z₁ désigne un atome d'hydrogène ou un radical alkyle linéaire ou ramifié en C1-C6, ou cyclique saturé ou insaturé en C3-C6, ledit radical étant éventuellement substitué par au moins un groupement choisi parmi =NH, -NH₂, -N(T)₂, =O, -OH, - OT, -SH, -ST, -CO₂T, phényle, phényle substitué par -OH ou -OT, 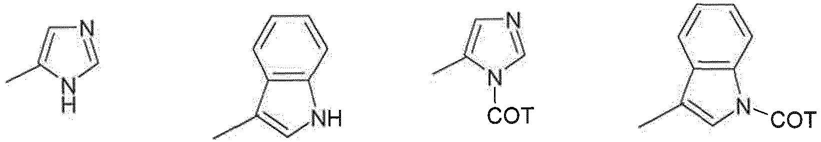 et/ou interrompu par un groupement -NH-, -N-(COT)-, ou -S- avec T désignant un radical alkyle linéaire ou ramifié en C1-C6, ou cyclique en C3-C6.
et Z₂ désigne un atome d'hydrogène ou un radical alkyle linéaire ou ramifié en C1-C6
**R_{c}** désigne :
- un atome d'hydrogène
- un radical alkyle linéaire en C1-C4, ou ramifié en C3-C4, ou un radical hydrocarboné insaturé linéaire ou ramifié en C3-C4, ledit radical étant éventuellement substitué par un groupement phényle.
**Rd** désigne :
- un atome d'hydrogène
- un radical alkyle linéaire en C1-C18, ou ramifié en C3-C18, ou un radical hydrocarboné insaturé linéaire ou ramifié en C3-C18, ledit radical étant éventuellement substitué par un groupe phényle
ainsi que leurs sels cosmétiquement acceptables, leurs solvates tels que les hydrates, et leurs stéréoisomères.

10. Composés C-xylosides de formule (IV) : dans laquelle, 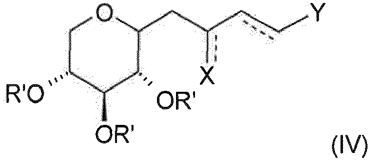
- les composés de formule (IV) sont des dérivés de xylose
- **Y** désigne un radical phényle éventuellement substitué par 1 à 5 groupements (ORₐ)
- **X** = -OH ; (=O)
- **R'** désigne :
* un radical alkyle linéaire ou ramifié, saturé ou insaturé en C1-C18, , ou cyclique saturé ou insaturé en C5 ou C6
* un radical acyle linéaire ou ramifié, saturé ou insaturé en C1-C18, ou cyclique saturé ou insaturé en C5 ou C6.
**Rₐ** désigne :
- un atome d'hydrogène
- un radical alkyle linéaire ou ramifié en C1-C4, ou un radical hydrocarboné insaturé linéaire ou ramifié en C3-C4
- un radical acyle linéaire ou ramifié en C1-C18 ou alcénylcarbonyle linéaire ou ramifié en C2-C18
Lorsque **Y** désigne un radical phényle substitué par 2 à 5 groupements (ORₐ), deux groupements adjacents ORₐ peuvent former ensemble un radical divalent-O-CH₂-O
sous réserve que lorsque X=OH, le composé ne comporte pas de double liaison éthylénique en position alpha du carbone portant cet OH,
ainsi que leurs sels cosmétiquement acceptables, leurs solvates tels que les hydrates, et leurs stéréoisomères,
à l'exception des composés suivants :

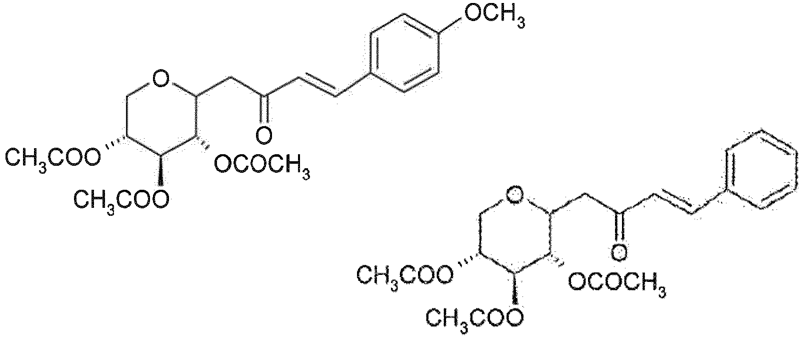

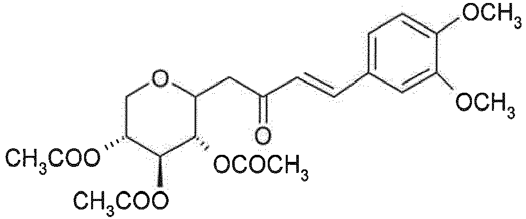

11. Composés C-xylosides de formule (V) : dans laquelle, 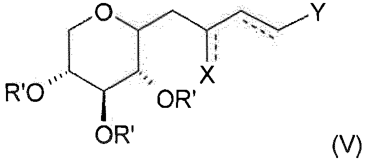
- les composés de formule (V) sont des dérivés de xylose
- **Y** désigne un radical phényle substitué par 1 à 5 groupements (ORₐ)
- **X** = -OH ; (=O)
- **R'** désigne un atome d'hydrogène
**Rₐ** désigne
- un radical alkyle linéaire ou ramifié en C1-C4, ou un radical hydrocarboné insaturé linéaire ou ramifié en C3-C4
- un radical acyle linéaire en C1-C18 ou ramifié en C3-C18 ou alcénylcarbonyle linéaire ou ramifié en C2-C18
sous réserve que lorsque X=OH, le composé ne comporte pas de double liaison éthylénique en position alpha du carbone portant cet OH.
Lorsque Y désigne un radical phényle substitué par 2 à 5 groupements (ORₐ), deux groupements adjacents ORₐ peuvent former ensemble un radical divalent-O-CH₂-O,
ainsi que leurs sels cosmétiquement acceptables, leurs solvates tels que les hydrates, et leurs stéréoisomères,
à l'exception du composé suivant :

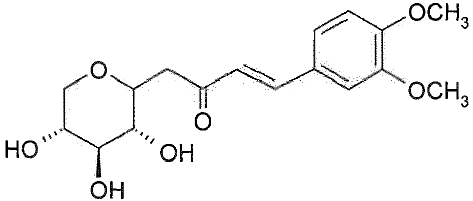

12. Composés C-xylosides de formules 5, 6, 10, 11, 12, 13, 14, 15, 16
Composé 5. (3R,4S,5R)-2-[2-(benzylamino)-4-(4-hydroxy-3-methoxyphenyl)butyl]tetrahydro-2H-pyran-3,4,5-triol 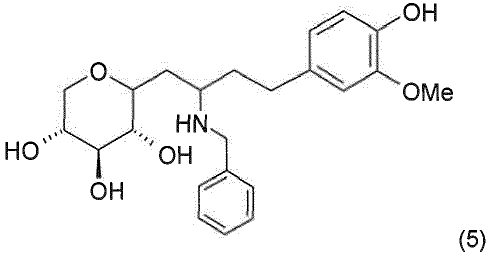
**Composé 6** : ethyl {[3-(4-hydroxy-3-methoxyphenyl)-1-{[(3R,4S,5R)-3,4,5-trihydroxytetrahydro-2H-pyran-2-yl]methyl}propyl]amino}(phenyl)acetate 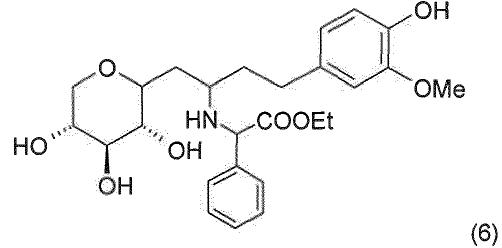
**Composé 10 :** (3R,4S,5R)-2-[2-hydroxy-4-(3-hydroxy-4-methoxyphenyl)butyl]tetrahydro-2H-pyran-3,4,5-triol 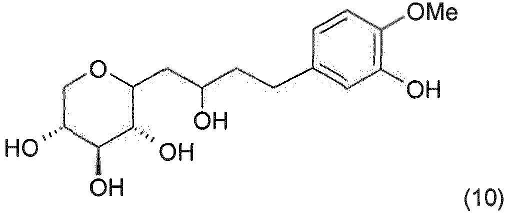
**Composé 11.** (3R,4S,5R)-2-[2-hydroxy-4-(2,4-di-hydroxy-phenyl)butyl]tetrahydro-2H-pyran-3,4,5-triol 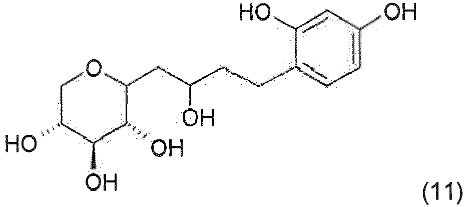
**Composé 12.** (3R,4S,5R)-2-[2-hydroxy-4-(3-ethoxy-4-hydroxy-phenyl)butyl]tetrahydro-2H-pyran-3,4,5-triol 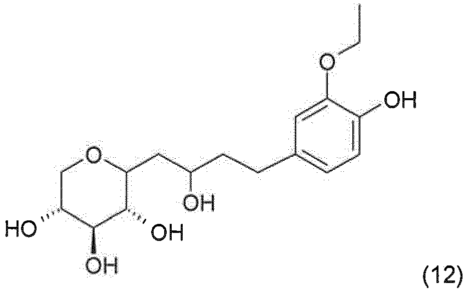
**Composé 13.** 5,9-anhydro-1,2,4-trideoxy-1-pyridin-3-yl-D-xylo-nonitol 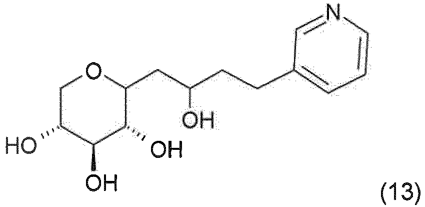
**Composé 14.** (3R,4S,5R)-2-[(2E)-2-(methoxyimino)-4-phenylbutyl]tetrahydro-2H-pyran-3,4,5-triol 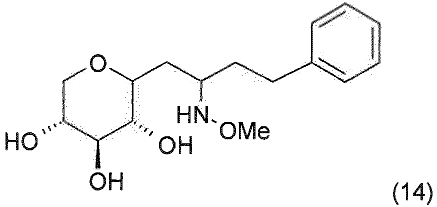
**Composé 15.** 5,9-anhydro-1,2,4-trideoxy-7-O-pentanoyl-1-phenyl-D-xylo-non-3-ulose 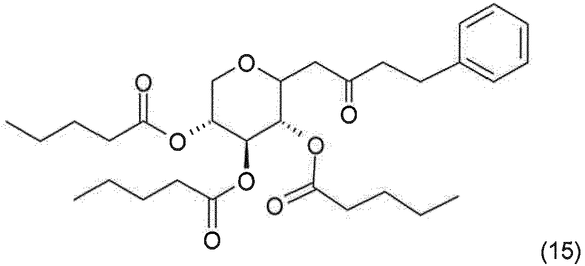
**Composé 16.** 5,9-anhydro-1,2,4-trideoxy-1-(3,4,5-trimethoxyphenyl)-D-xylo-nonitol

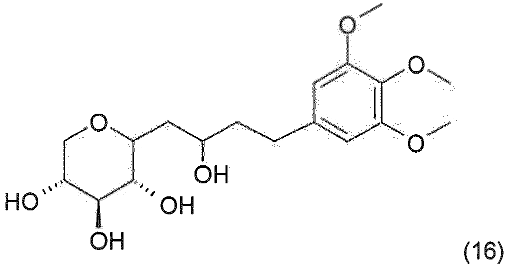

13. Composition comprenant, dans un milieu physiologiquement acceptable, au moins un composé C-xylosides de formule (II ) selon la revendication 8.

14. Composition comprenant, dans un milieu physiologiquement acceptable, au moins un composé C-xylosides de formule (III) selon la revendication 9.

15. Composition comprenant, dans un milieu physiologiquement acceptable, au moins un composé C-xylosides de formule (IV) selon la revendication 10.

16. Composition comprenant, dans un milieu physiologiquement acceptable, au moins un composé C-xylosides de formule (V) selon la revendication 11.

17. Composition comprenant, dans un milieu physiologiquement acceptable, au moins un composé C-xylosides selon la revendication 12.

## Patentansprüche

1. Kosmetisches Verfahren zur Depigmentierung, Aufhellung und/oder Bleichung der Haut, des Körperhaars oder des Kopfhaars, umfassend das Aufbringen einer Zusammensetzung, die in einem physiologisch unbedenklichen Medium mindestens eine Verbindung der Formel (I): 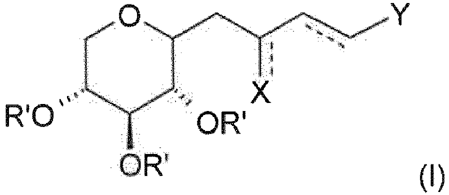 in der
- es sich bei den Verbindungen der Formel (I) um Xylosederivate handelt,
- Y für einen Phenylrest oder einen Heterocyclus, die gegebenenfalls durch 1 bis 5 Gruppen (ORₐ) substituiert sind, steht,
- X = -OR' ; (=O) ; NR_{b}R_{c}; NHOR_{d},
- R' für:
* ein Wasserstoffatom,
* einen linearen und gesättigten C1-C18-Alkylrest,
* einen linearen und ungesättigten C2-C18-Alkylrest,
* einen gesättigten oder ungesättigten, verzweigten C3-C18-Alkylrest,
* einen gesättigten oder ungesättigten cyclischen C5- oder C6-Rest,
* einen gesättigten oder ungesättigten, linearen oder verzweigten C2-C18-Acylrest oder einen gesättigten oder ungesättigten cyclischen C5- oder C6-Acylrest
steht,
Rₐ für:
- ein Wasserstoffatom,
- einen linearen oder verzweigten C1-C4-Alkylrest oder einen linearen oder verzweigten ungesättigten C3-C4-Kohlenwasserstoffrest,
- einen linearen oder verzweigten C2-C18-Acylrest oder einen linearen oder verzweigten C2-C18-Alkenylcarbonylrest
steht,
dann, wenn Y für einen Phenylrest oder einen Heterocyclus, der durch 2 bis 5 Gruppen (ORₐ) substituiert ist, steht, zwei benachbarte Gruppen ORₐ zusammen einen zweiwertigen Rest -O-CH₂-O bilden können,
mit der Maßgabe, dass im Fall von X = OH die Verbindung keine ethylenische Doppelbindung in alpha-Position zu dem dieses OH tragenden Kohlenstoff enthält,
R_{b} für:
- ein Wasserstoffatom,
- einen linearen C2-C18- oder verzweigten C3-C18-Alkylrest oder einen linearen oder verzweigten ungesättigten C3-C4-Kohlenwasserstoffrest oder einen Rest -CH(Z₁)-CO₂Z₂, in dem Z₁ für ein Wasserstoffatom oder einen linearen oder verzweigten C1-C6-Alkylrest oder einen gesättigten oder ungesättigten cyclischen C3-C6-Alkylrest steht, wobei der Rest gegebenenfalls durch mindestens eine Gruppe, die aus =NH, -NH₂, -N(T)₂, =O, -OH, -OT, -SH, -ST, -CO₂T, Phenyl, Phenyl, das durch -OH oder -OT substituiert ist, 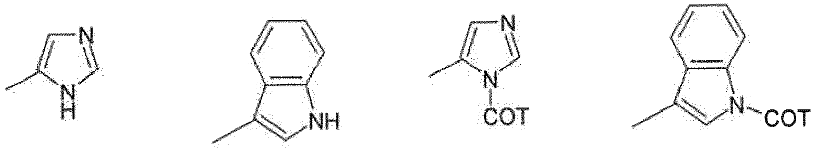
ausgewählt ist, substituiert und/oder durch eine Gruppe -NH-, -N-(COT)- oder -S- unterbrochen ist, wobei T für einen linearen oder verzweigten C1-C6-Alkylrest oder einen cyclischen C3-C6-Alkylrest steht,
und Z₂ für ein Wasserstoffatom oder einen linearen oder verzweigten C1-C6-Alkylrest steht, steht,
R_{c} für:
- ein Wasserstoffatom,
- einen linearen C1-C4- oder verzweigten C3-C4-Alkylrest oder einen linearen oder verzweigten ungesättigten C3-C4-Kohlenwasserstoffrest, wobei der Rest gegebenenfalls durch eine Phenylgruppe substituiert ist,
steht,
R_{d} für:
- ein Wasserstoffatom,
- einen linearen C1-C18- oder verzweigten C3-C18-Alkylrest oder einen linearen oder verzweigten ungesättigten C3-C18-Kohlenwasserstoffrest, wobei der Rest gegebenenfalls durch eine Phenylgruppe substituiert ist,
steht,
sowie die kosmetisch unbedenklichen Salze davon, Solvate davon wie Hydrate und Stereoisomere davon umfasst, auf die Haut, das Körperhaar oder das Kopfhaar.

2. Verfahren nach dem vorhergehenden Anspruch, **dadurch gekennzeichnet, dass**:
- es sich bei den Verbindungen der Formel (I) um Xylosederivate handelt,
- Y für einen Phenylrest oder einen Heterocyclus, die gegebenenfalls durch 1 bis 5 Gruppen (ORₐ) substituiert sind, steht,
- X = -OR' ; (=O) ; NR_{b}R_{c}; NHOR_{d},
- R' für:
* ein Wasserstoffatom,
* einen gesättigten oder ungesättigten, linearen oder verzweigten C1-C12-Alkylrest oder einen gesättigten cyclischen C5- oder C6-Alkylrest,
* einen gesättigten oder ungesättigten, linearen oder verzweigten C2-C6-Acylrest oder einen gesättigten cyclischen C5- oder C6-Acylrest
steht,
Rₐ für:
- ein Wasserstoffatom,
- einen linearen oder verzweigten C1-C4-Alkylrest,
- einen linearen oder verzweigten C2-C6-Acylrest steht,
dann, wenn Y für einen Phenylrest oder einen Heterocyclus, der durch 2 bis 5 Gruppen (ORₐ) substituiert ist, steht, zwei benachbarte Gruppen ORₐ zusammen einen zweiwertigen Rest -O-CH₂-O bilden können,
mit der Maßgabe, dass im Fall von X = OH die Verbindung keine ethylenische Doppelbindung in alpha-Position zu dem dieses OH tragenden Kohlenstoff enthält,
R_{b} für:
- ein Wasserstoffatom,
- einen linearen C2-C12- oder verzweigten C3-C12-Alkylrest oder einen Rest -CH(Z₁)-CO₂Z₂, in dem Z₁ für ein Wasserstoffatom oder einen linearen oder verzweigten C1-C6-Alkylrest oder einen gesättigten oder ungesättigten cyclischen C3-C6-Alkylrest steht, wobei der Rest gegebenenfalls durch mindestens eine Gruppe, die aus =NH, -NH₂, -N(T)₂, =0, -OH, -OT, -SH, -ST, -CO₂T, Phenyl, Phenyl, das durch -OH oder -OT substituiert ist, 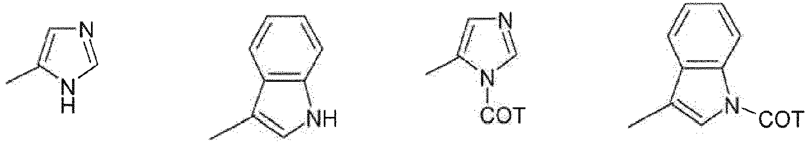
ausgewählt ist, substituiert und/oder durch eine Gruppe -NH-, -N-(COT)- oder -S- unterbrochen ist, wobei T für einen linearen C1-C6-Alkylrest oder einen cyclischen C5-C6-Alkylrest steht,
und Z₂ für ein Wasserstoffatom oder einen linearen oder verzweigten C1-C6-Alkylrest steht, steht,
R_{c} für:
- ein Wasserstoffatom,
- einen linearen C1-C4- oder verzweigten C3-C4-Alkylrest oder einen linearen oder verzweigten ungesättigten C3-C4-Kohlenwasserstoffrest, wobei der Rest gegebenenfalls durch eine Phenylgruppe substituiert ist,
steht,
R_{d} für:
- ein Wasserstoffatom,
- einen linearen C1-C12- oder verzweigten C3-C12-Alkylrest, wobei der Rest gegebenenfalls durch eine Phenylgruppe substituiert ist,
steht,
sowie die kosmetisch unbedenklichen Salze davon, Solvate davon wie Hydrate und Stereoisomere davon.

3. Verfahren nach einem der vorhergehenden Ansprüche, **dadurch gekennzeichnet, dass**:
- es sich bei den Verbindungen der Formel (I) um Xylosederivate handelt,
- Y für einen Phenylrest oder einen Heterocyclus, die gegebenenfalls durch 1 bis 3 Gruppen (ORₐ) substituiert sind, steht,
- X = -OR' ; (=O) ; NR_{b}R_{c}; NHOR_{d},
- R' für:
* ein Wasserstoffatom,
* einen gesättigten oder ungesättigten, linearen oder verzweigten C1-C4-Alkylrest
* einen linearen oder verzweigten C2-C6-Acylrest
steht,
Rₐ für:
- ein Wasserstoffatom,
- einen linearen oder verzweigten C1-C4-Alkylrest,
- einen linearen oder verzweigten C2-C6-Acylrest steht,
dann, wenn Y für einen Phenylrest oder einen Heterocyclus, der durch 2 oder 3 Gruppen (ORₐ) substituiert ist, steht, zwei benachbarte Gruppen ORₐ zusammen einen zweiwertigen Rest -O-CH₂-O bilden können,
mit der Maßgabe, dass im Fall von X = OH die Verbindung keine ethylenische Doppelbindung in alpha-Position zu dem dieses OH tragenden Kohlenstoff enthält,
R_{b} für:
- ein Wasserstoffatom,
- einen linearen C2-C8- oder verzweigten C3-C8-Alkylrest oder einen Rest -CH(Z₁)-CO₂Z₂, in dem Z₁ für ein Wasserstoffatom oder einen linearen oder verzweigten C1-C6-Alkylrest oder einen gesättigten oder ungesättigten cyclischen C3-C6-Alkylrest steht, wobei der Rest gegebenenfalls durch mindestens eine Gruppe, die aus =NH, -NH₂, -N(T)₂, =0, -OH, -OT, -SH, -ST, -CO₂T, Phenyl, Phenyl, das durch -OH oder -OT substituiert ist, 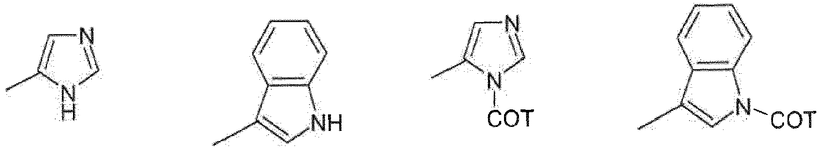
ausgewählt ist, substituiert und/oder durch eine Gruppe -NH-, -N-(COT)- oder -S- unterbrochen ist,
wobei T für einen linearen C1-C6-Alkylrest oder einen cyclischen C5-C6-Alkylrest steht,
und Z₂ für ein Wasserstoffatom oder einen linearen oder verzweigten C1-C6-Alkylrest steht, steht,
R_{c} für:
- ein Wasserstoffatom,
- einen linearen C1-C4- oder verzweigten C3-C4-Alkylrest, der gegebenenfalls durch eine Phenylgruppe substituiert ist,
steht,
R_{d} für:
- ein Wasserstoffatom,
- einen linearen C1-C4- oder verzweigten C3-C4-Alkylrest, wobei der Rest gegebenenfalls durch eine Phenylgruppe substituiert ist,
steht,
sowie die kosmetisch unbedenklichen Salze davon, Solvate davon wie Hydrate und Stereoisomere davon.

4. Verfahren nach einem der vorhergehenden Ansprüche, **dadurch gekennzeichnet, dass** die Verbindung der Formel (I) ausgewählt wird aus:
Verbindung 1. (3R,4S,5R)-2-[2-Hydroxy-4-(4-hydroxy-3-methoxyphenyl)butyl]tetrahydro-2H-pyran-3,4,5-triol 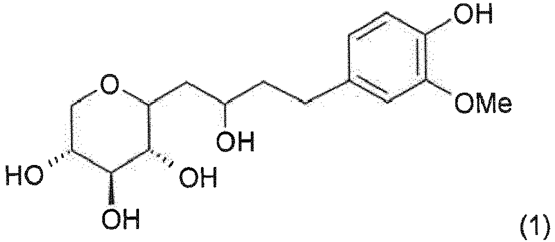
Verbindung 2. 4-(4-Hydroxy-3-methoxyphenyl)-1-[(3R,4S,5R)-3,4,5-trihydroxytetrahydro-2H-pyran-2-yl]butan-2-on 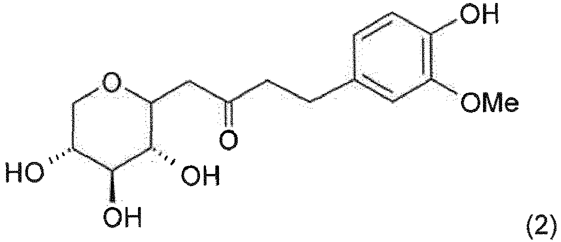
Verbindung 3. (3R,4S,5R)-2-[2-Hydroxy-4-(4-hydroxyphenyl)butyl]tetrahydro-2H-pyran-3,4,5-triol 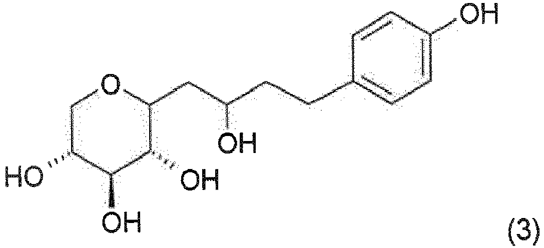
Verbindung 4. 4-(4-Hydroxyphenyl)-1-[(3R,4S,5R)-3,4,5-trihydroxytetrahydro-2H-pyran-2-yl]butan-2-on 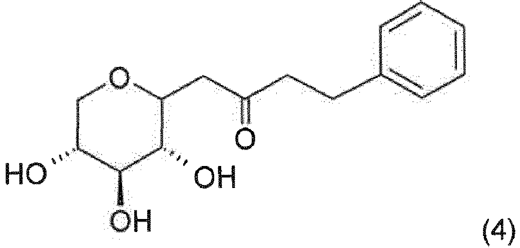
Verbindung 5. (3R,4S,5R)-2-[2-(Benzylamino)-4-(4-hydroxy-3-methoxyphenyl)butyl]tetrahydro-2H-pyran-3,4,5-triol 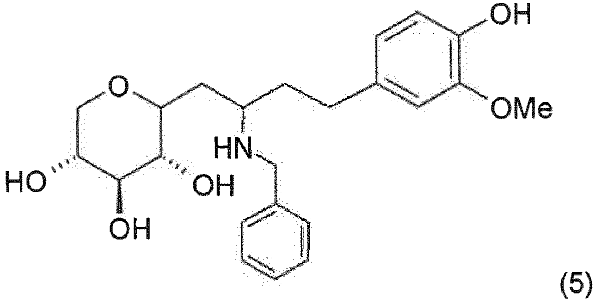
Verbindung 6. {[3-(4-Hydroxy-3-methoxyphenyl)-1-{[(3R,4S,5R)-3,4,5-trihydroxytetrahydro-2H-pyran-2-yl]methyl}propyl]amino}(phenyl) essigsäureethylester 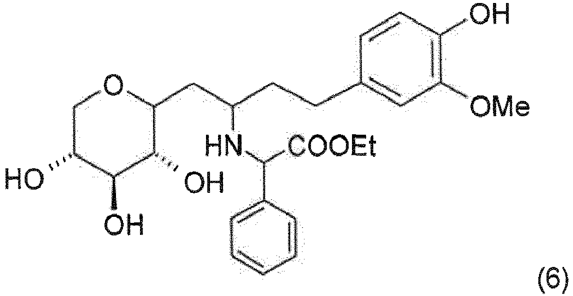
Verbindung 7. (3E)-4-Phenyl-1-[(3R,4S,5R)-3,4,5-trihydroxytetrahydro-2H-pyran-2-yl]but-3-en-2-on 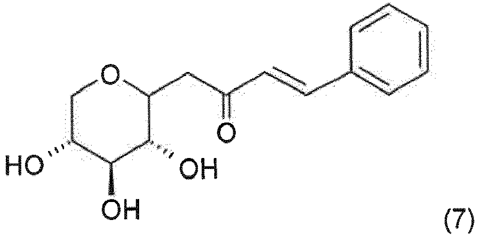
Verbindung 8. (3E)-4-(4-Hydroxy-3,5-dimethoxyphenyl)-1-[(3R,4S,5R)-3,4,5-trihydroxytetrahydro-2H-pyran-2-yl]but-3-en-2-on 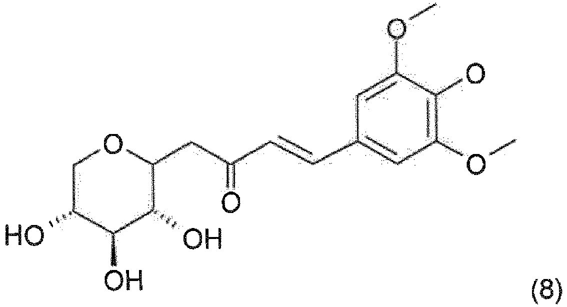
Verbindung 9. (3R,4S,5R)-2-[2-Hydroxy-4-(2-hydroxyphenyl)butyl]tetrahydro-2H-pyran-3,4,5-triol 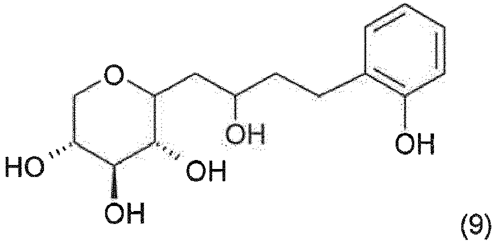
Verbindung 10: (3R,4S,5R)-2-[2-Hydroxy-4-(3-hydroxy-4-methoxyphenyl)butyl]tetrahydro-2H-pyran-3,4,5-triol 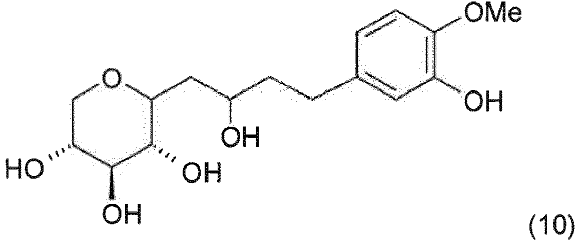
Verbindung 11. (3R,4S,5R)-2-[2-Hydroxy-4-(2,4-di-hydroxyphenyl)butyl]tetrahydro-2H-pyran-3,4,5-triol 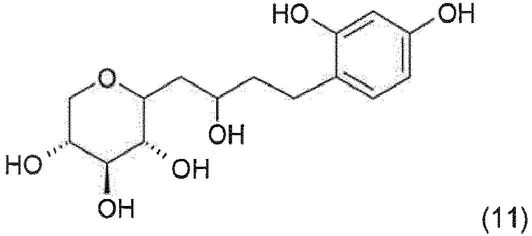
Verbindung 12. (3R,4S,5R)-2-[2-Hydroxy-4-(3-ethoxy-4-hydroxyphenyl)butyl]tetrahydro-2H-pyran-3,4,5-triol 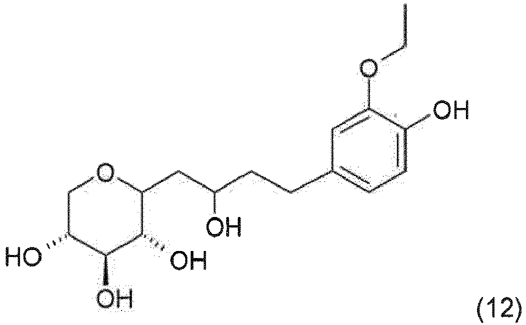
Verbindung 13. 5,9-Anhydro-1,2,4-tridesoxy-1-pyridin-3-yl-D-xylononitol 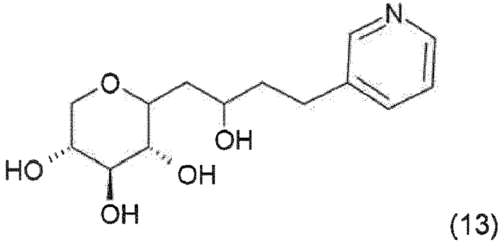
Verbindung 14. (3R,4S,5R)-2-[(2E)-2-(Methoxyimino)-4-phenylbutyl]tetrahydro-2H-pyran-3,4,5-triol 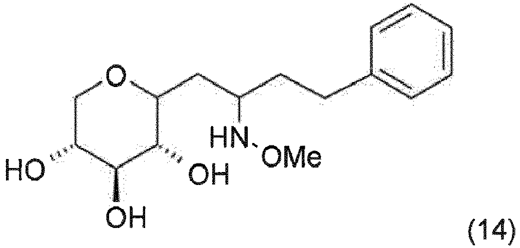
Verbindung 15. 5,9-Anhydro-1,2,4-tridesoxy-7-O-pentanoyl-1-phenyl-D-xylonon-3-ulos 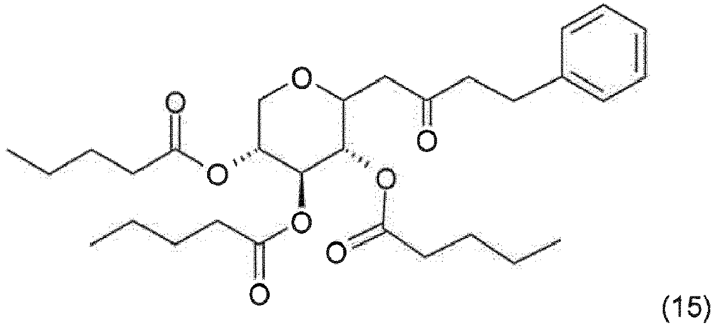
Verbindung 16. 5,9-Anhydro-1,2,4-tridesoxy-1-(3,4,5-trimethoxyphenyl)-D-xylononitol

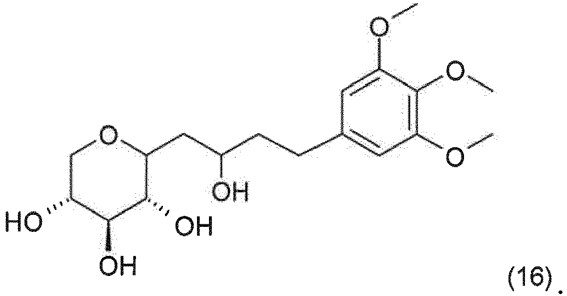

5. Verfahren nach einem der vorhergehenden Ansprüche, **dadurch gekennzeichnet, dass** die Verbindung der Formel (I) ausgewählt ist aus:
Verbindung 11. (3R,4S,5R)-2-[2-Hydroxy-4-(2,4-di-hydroxyphenyl)butyl]tetrahydro-2H-pyran-3,4,5-triol 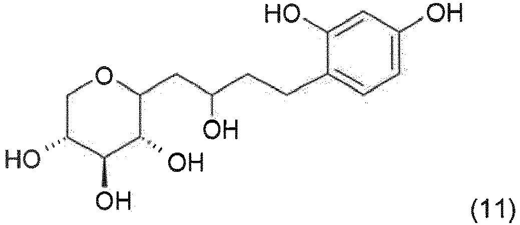
Verbindung 12. (3R,4S,5R)-2-[2-Hydroxy-4-(3-ethoxy-4-hydroxyphenyl)butyl]tetrahydro-2H-pyran-3,4,5-triol

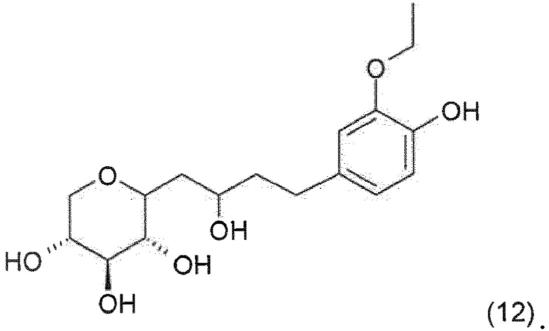

6. Verfahren nach einem der vorhergehenden Ansprüche, **dadurch gekennzeichnet, dass** die Verbindung der Formel (I) in einer Menge im Bereich von 0,001 bis 10 Gew.-%, bezogen auf das Gesamtgewicht der Zusammensetzung, vorzugsweise im Bereich von 0,01 bis 5 Gew.-%, insbesondere von 0,1 bis 2%, vorliegt.

7. Kosmetische Verwendung einer Verbindung der Formel (I) gemäß einem der Ansprüche 1 bis 6 als Mittel zur Bleichung, Aufhellung und/oder Depigmentierung der Haut, des Körperhaars oder des Kopfhaars.

8. C-Xylosid-Verbindungen der Formel (II): 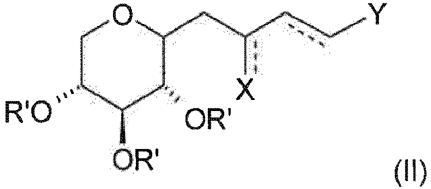 in der
- es sich bei den Verbindungen der Formel (II) um Xylosederivate handelt,
- Y für einen Heterocyclus, der gegebenenfalls durch 1 bis 5 Gruppen (ORₐ) substituiert ist, steht,
- X = -OR' ; (=O) ; NR_{b}R_{c}; NHOR_{d},
R' für:
* ein Wasserstoffatom,
* einen linearen und gesättigten C1-C18-Alkylrest,
* einen linearen und ungesättigten C2-C18-Alkylrest,
* einen gesättigten oder ungesättigten, verzweigten C3-C18-Alkylrest,
* einen gesättigten oder ungesättigten cyclischen C5- oder C6-Rest,
* einen gesättigten oder ungesättigten, linearen oder verzweigten C2-C18-Acylrest oder einen gesättigten oder ungesättigten cyclischen C5- oder C6-Acylrest steht,
Rₐ für:
- ein Wasserstoffatom,
- einen linearen oder verzweigten C1-C4-Alkylrest oder einen linearen oder verzweigten ungesättigten C3-C4-Kohlenwasserstoffrest,
- einen linearen oder verzweigten C2-C18-Acylrest oder einen linearen oder verzweigten C2-C18-Alkenylcarbonylrest
steht,
R_{b} für:
- ein Wasserstoffatom,
- einen linearen C2-C18- oder verzweigten C3-C18-Alkylrest oder einen linearen oder verzweigten ungesättigten C3-C4-Kohlenwasserstoffrest oder einen Rest -CH(Z₁)-CO₂Z₂, in dem Z₁ für ein Wasserstoffatom oder einen linearen oder verzweigten C1-C6-Alkylrest oder einen gesättigten oder ungesättigten cyclischen C3-C6-Alkylrest steht, wobei der Rest gegebenenfalls durch mindestens eine Gruppe, die aus =NH, -NH₂, -N(T)₂, =O, -OH, -OT, -SH, -ST, -CO₂T, Phenyl, Phenyl, das durch -OH oder -OT substituiert ist, 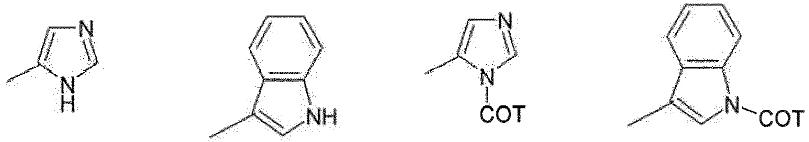 ausgewählt ist, substituiert und/oder durch eine Gruppe -NH-, -N-(COT)- oder -S- unterbrochen ist, wobei T für einen linearen oder verzweigten C1-C6-Alkylrest oder einen cyclischen C3-C6-Alkylrest steht,
und Z₂ für ein Wasserstoffatom oder einen linearen oder verzweigten C1-C6-Alkylrest steht, steht,
R_{c} für:
- ein Wasserstoffatom,
- einen linearen C1-C4- oder verzweigten C3-C4-Alkylrest oder einen linearen oder verzweigten ungesättigten C3-C4-Kohlenwasserstoffrest, wobei der Rest gegebenenfalls durch eine Phenylgruppe substituiert ist,
steht,
R_{d} für:
- ein Wasserstoffatom,
- einen linearen C1-C18- oder verzweigten C3-C18-Alkylrest oder einen linearen oder verzweigten ungesättigten C3-C18-Kohlenwasserstoffrest, wobei der Rest gegebenenfalls durch eine Phenylgruppe substituiert ist,
steht,
sowie die kosmetisch unbedenklichen Salze davon, Solvate davon wie Hydrate und Stereoisomere davon.

9. C-Xylosid-Verbindungen der Formel (III): 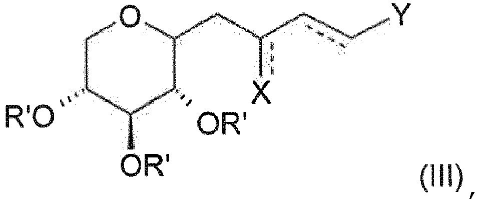 in der
- es sich bei den Verbindungen der Formel (III) um Xylosederivate handelt,
- Y für einen Phenylrest, der gegebenenfalls durch 1 bis 5 Gruppen (ORₐ) substituiert ist, steht,
- X = -OR'' ; NR_{b}R_{c}; NHOR_{d},
- R' für:
* ein Wasserstoffatom,
* einen gesättigten oder ungesättigten, linearen oder verzweigten C1-C18-oder gesättigten oder ungesättigten cyclischen C5- oder C6-Alkylrest,
* einen gesättigten oder ungesättigten, linearen oder verzweigten C1-C18- oder gesättigten oder ungesättigten cyclischen C5- oder C6-Acylrest
steht,
- R" für:
* einen gesättigten oder ungesättigten, linearen oder verzweigten C1-C18-oder gesättigten oder ungesättigten cyclischen C5- oder C6-Alkylrest,
* einen gesättigten oder ungesättigten, linearen oder verzweigten C1-C18- oder gesättigten oder ungesättigten cyclischen C5- oder C6-Acylrest
steht,
Rₐ für:
- ein Wasserstoffatom,
- einen linearen oder verzweigten C1-C4-Alkylrest oder einen linearen oder verzweigten ungesättigten C3-C4-Kohlenwasserstoffrest,
- einen linearen oder verzweigten C1-C18-Acylrest oder einen linearen oder verzweigten C2-C18-Alkenylcarbonylrest
steht,
dann, wenn Y für einen Phenylrest, der durch 2 bis 5 Gruppen (ORₐ) substituiert ist, steht, zwei benachbarte Gruppen ORₐ zusammen einen zweiwertigen Rest -O-CH₂-O bilden können,
R_{b} für:
- ein Wasserstoffatom,
- einen linearen C2-C18- oder verzweigten C3-C18-Alkylrest oder einen linearen oder verzweigten ungesättigten C3-C4-Kohlenwasserstoffrest oder einen Rest -CH(Z₁)-CO₂Z₂, in dem
Z₁ für ein Wasserstoffatom oder einen linearen oder verzweigten C1-C6-Alkylrest oder einen gesättigten oder ungesättigten cyclischen C3-C6-Alkylrest steht, wobei der Rest gegebenenfalls durch mindestens eine Gruppe, die aus =NH, -NH₂, -N(T)₂, =O, -OH, -OT, -SH, -ST, -CO₂T, Phenyl, Phenyl, das durch -OH oder -OT substituiert ist, 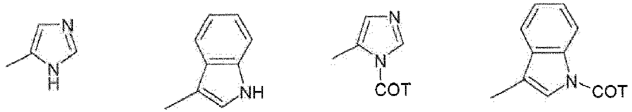
ausgewählt ist, substituiert und/oder durch eine Gruppe -NH-, -N-(COT)- oder -S- unterbrochen ist, wobei T für einen linearen oder verzweigten C1-C6-Alkylrest oder einen cyclischen C3-C6-Alkylrest steht,
und Z₂ für ein Wasserstoffatom oder einen linearen oder verzweigten C1-C6-Alkylrest steht, steht,
R_{c} für:
- ein Wasserstoffatom,
- einen linearen C1-C4- oder verzweigten C3-C4-Alkylrest oder einen linearen oder verzweigten ungesättigten C3-C4-Kohlenwasserstoffrest, wobei der Rest gegebenenfalls durch eine Phenylgruppe substituiert ist,
steht,
R_{d} für:
- ein Wasserstoffatom,
- einen linearen C1-C18- oder verzweigten C3-C18-Alkylrest oder einen linearen oder verzweigten ungesättigten C3-C18-Kohlenwasserstoffrest, wobei der Rest gegebenenfalls durch eine Phenylgruppe substituiert ist,
steht,
sowie die kosmetisch unbedenklichen Salze davon, Solvate davon wie Hydrate und Stereoisomere davon.

10. C-Xylosid-Verbindungen der Formel (IV): 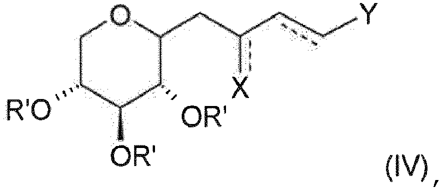 in der
- es sich bei den Verbindungen der Formel (IV) um Xylosederivate handelt,
- Y für einen Phenylrest, der gegebenenfalls durch 1 bis 5 Gruppen (ORₐ) substituiert ist, steht,
- X = -OH; (=O),
- R' für:
* einen gesättigten oder ungesättigten, linearen oder verzweigten C1-C18-oder gesättigten oder ungesättigten cyclischen C5- oder C6-Alkylrest,
* einen gesättigten oder ungesättigten, linearen oder verzweigten C1-C18- oder gesättigten oder ungesättigten cyclischen C5- oder C6-Acylrest
steht,
Rₐ für:
- ein Wasserstoffatom,
- einen linearen oder verzweigten C1-C4-Alkylrest oder einen linearen oder verzweigten ungesättigten C3-C4-Kohlenwasserstoffrest,
- einen linearen oder verzweigten C1-C18-Acylrest oder einen linearen oder verzweigten C2-C18-Alkenylcarbonylrest
steht,
dann, wenn Y für einen Phenylrest, der durch 2 bis 5 Gruppen (ORₐ) substituiert ist, steht, zwei benachbarte Gruppen ORₐ zusammen einen zweiwertigen Rest -O-CH₂-O bilden können,
mit der Maßgabe, dass im Fall von X = OH die Verbindung keine ethylenische Doppelbindung in alpha-Position zu dem dieses OH tragenden Kohlenstoff enthält,
sowie die kosmetisch unbedenklichen Salze davon, Solvate davon wie Hydrate und Stereoisomere davon, mit Ausnahme der folgenden Verbindungen:

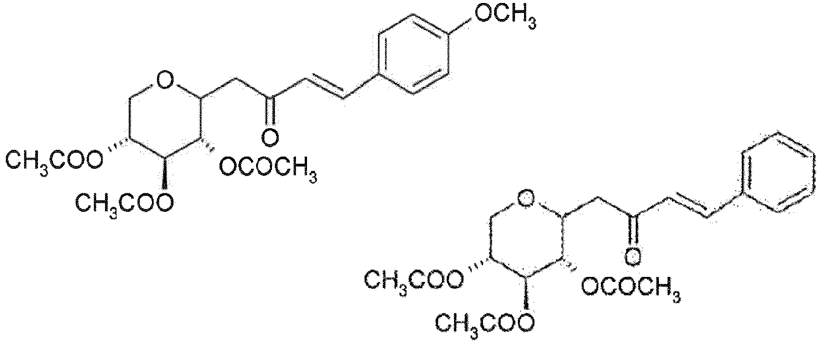

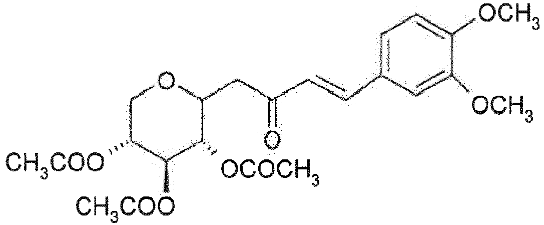

11. C-Xylosid-Verbindungen der Formel (V): 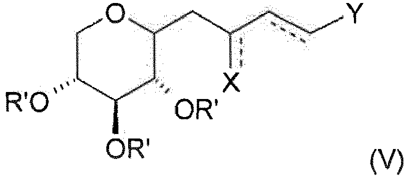 in der
- es sich bei den Verbindungen der Formel (V) um Xylosederivate handelt,
- Y für einen Phenylrest, der durch 1 bis 5 Gruppen (ORₐ) substituiert ist, steht,
- X = -OH; (=O),
- R' für ein Wasserstoffatom steht,
Rₐ für:
- einen linearen oder verzweigten C1-C4-Alkylrest oder einen linearen oder verzweigten ungesättigten C3-C4-Kohlenwasserstoffrest,
- einen linearen C1-C18- oder verzweigten C3-C18-Acylrest oder einen linearen oder verzweigten C2-C18-Alkenylcarbonylrest
steht,
mit der Maßgabe, dass im Fall von X = OH die Verbindung keine ethylenische Doppelbindung in alpha-Position zu dem dieses OH tragenden Kohlenstoff enthält,
dann, wenn Y für einen Phenylrest, der durch 2 bis 5 Gruppen (ORₐ) substituiert ist, steht, zwei benachbarte Gruppen ORₐ zusammen einen zweiwertigen Rest -O-CH₂-O bilden können,
sowie die kosmetisch unbedenklichen Salze davon, Solvate davon wie Hydrate und Stereoisomere davon, mit Ausnahme der folgenden Verbindung:

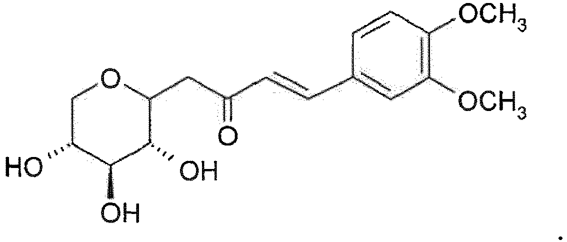

12. C-Xylosid-Verbindungen der Formeln 5, 6, 10, 11, 12, 13, 14, 15, 16
Verbindung 5. (3R,4S,5R)-2-[2-(Benzylamino)-4-(4-hydroxy-3-methoxyphenyl)butyl]tetrahydro-2H-pyran-3,4,5-triol 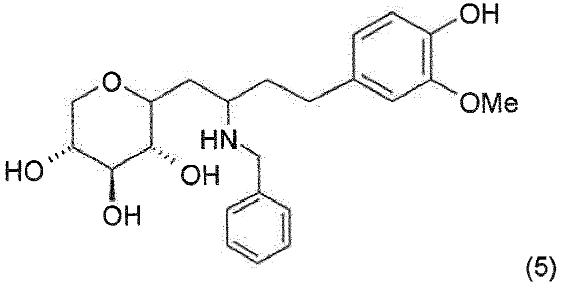
Verbindung 6: {[3-(4-Hydroxy-3-methoxyphenyl)-1-{[(3R,4S,5R)-3,4,5-trihydroxytetrahydro-2H-pyran-2-yl]methyl}propyl]amino}(phenyl) essigsäureethylester 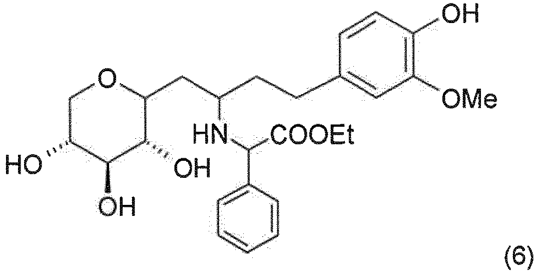
Verbindung 10: (3R,4S,5R)-2-[2-Hydroxy-4-(3-hydroxy-4-methoxyphenyl)butyl]tetrahydro-2H-pyran-3,4,5-triol 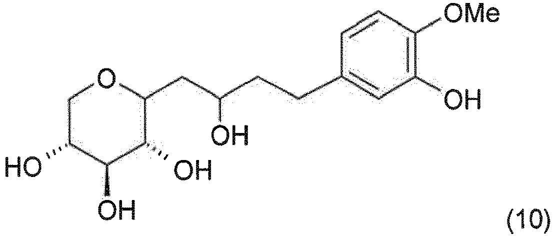
Verbindung 11. (3R,4S,5R)-2-[2-Hydroxy-4-(2,4-di-hydroxyphenyl)butyl]tetrahydro-2H-pyran-3,4,5-triol 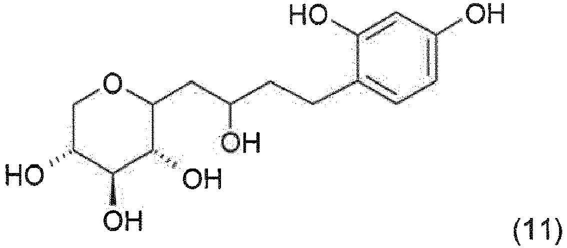
Verbindung 12. (3R,4S,5R)-2-[2-Hydroxy-4-(3-ethoxy-4-hydroxyphenyl)butyl]tetrahydro-2H-pyran-3,4,5-triol 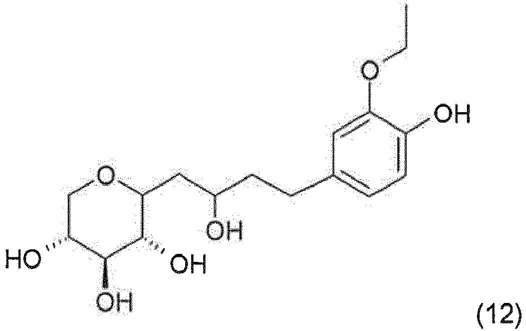
Verbindung 13. 5,9-Anhydro-1,2,4-tridesoxy-1-pyridin-3-yl-D-xylononitol 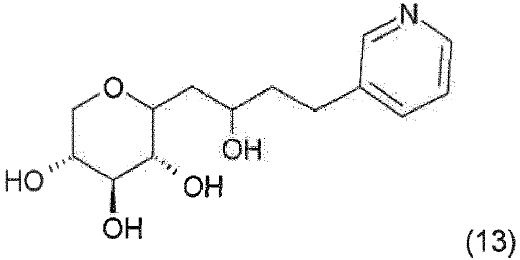
Verbindung 14. (3R,4S,5R)-2-[(2E)-2-(Methoxyimino)-4-phenylbutyl]tetrahydro-2H-pyran-3,4,5-triol 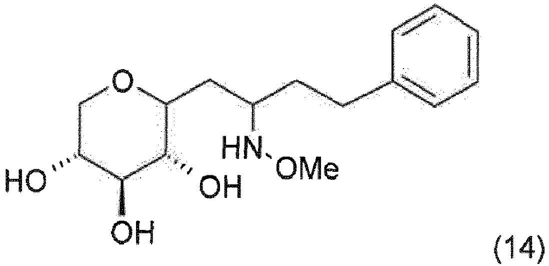
Verbindung 15. 5,9-Anhydro-1,2,4-tridesoxy-7-O-pentanoyl-1-phenyl-D-xylonon-3-ulos 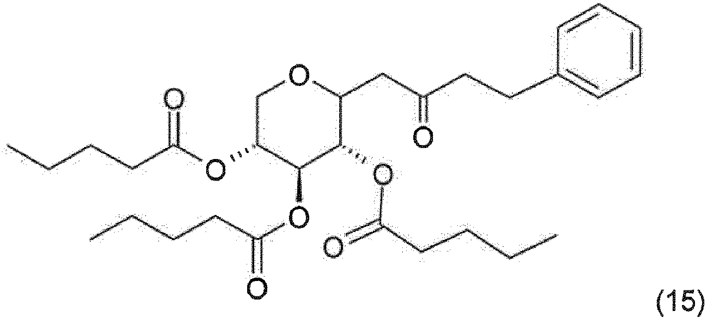
Verbindung 16. 5,9-Anhydro-1,2,4-tridesoxy-1-(3,4,5-trimethoxyphenyl)-D-xylononitol

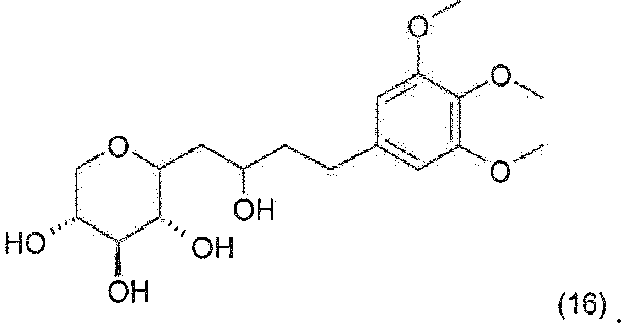

13. Zusammensetzung, die in einem physiologisch unbedenklichen Medium mindestens eine C-Xylosid-Verbindung der Formel (II) nach Anspruch 8 umfasst.

14. Zusammensetzung, die in einem physiologisch unbedenklichen Medium mindestens eine C-Xylosid-Verbindung der Formel (III) nach Anspruch 9 umfasst.

15. Zusammensetzung, die in einem physiologisch unbedenklichen Medium mindestens eine C-Xylosid-Verbindung der Formel (IV) nach Anspruch 10 umfasst.

16. Zusammensetzung, die in einem physiologisch unbedenklichen Medium mindestens eine C-Xylosid-Verbindung der Formel (V) nach Anspruch 11 umfasst.

17. Zusammensetzung, die in einem physiologisch unbedenklichen Medium mindestens eine C-Xylosid-Verbindung nach Anspruch 12 umfasst.

## Claims

1. Cosmetic process for depigmenting, lightening and/or bleaching the skin, bodily hairs or head hair, comprising the application to the skin, bodily hairs or head hair of a composition comprising, in a physiologically acceptable medium, at least one compound of formula (I) : 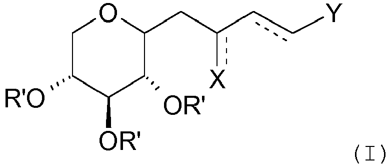 in which:
- the compounds of formula (I) are xylose derivatives
- Y denotes a phenyl radical or a heterocycle, optionally substituted with 1 to 5 groups (ORₐ)
- X = -OR' ; (=O) ; NR_{b}R_{c}; NHOR_{d}
- R' denotes:
* a hydrogen atom,
* a saturated linear C1-C18 alkyl radical,
* an unsaturated linear C2-C18 alkyl radical,
* a saturated or unsaturated branched C3-C18 alkyl radical,
* a saturated or unsaturated cyclic C5 or C6 radical,
* a linear or branched, saturated or unsaturated C2-C18, or saturated or unsaturated cyclic C5 or C6 acyl radical.
**Rₐ** denotes:
- a hydrogen atom
- a linear or branched C1-C4 alkyl radical, or a linear or branched unsaturated C3-C4 hydrocarbon-based radical
- a linear or branched C2-C18 acyl or linear or branched C2-C18 alkenylcarbonyl radical
- when Y denotes a phenyl radical or a heterocycle substituted with 2 to 5 groups (ORₐ), two adjacent groups ORₐ may together form a divalent radical -O-CH₂-O with the proviso that when X = OH, the compound does not comprise an ethylenic double bond alpha to the carbon bearing this OH.
**R_{b}** denotes:
- a hydrogen atom
- a linear C2-C18 or branched C3-C18 alkyl radical, or a linear or branched unsaturated C3-C4 hydrocarbon-based radical or a radical -CH(Z₁)-CO₂Z₂ in which
Z₁ denotes a hydrogen atom or a linear or branched C1-C6, or saturated or unsaturated cyclic C3-C6 alkyl radical, said radical being optionally substituted with at least one group chosen from =NH, -NH₂, -N(T)₂, =O,-OH, -OT, -SH, -ST, -CO₂T, phenyl, phenyl substituted with -OH or -OT, 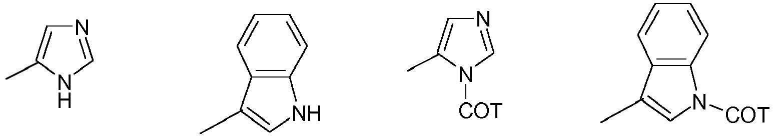 and/or interrupted with a group -NH-, -N-(COT)- or -S-with T denoting a linear or branched C1-C6 or cyclic C3-C6 alkyl radical.
and Z₂ denotes a hydrogen atom or a linear or branched C1-C6 alkyl radical
**R_{c}** denotes:
- a hydrogen atom
- a linear C1-C4 or branched C3-C4 alkyl radical, or a linear or branched unsaturated C3-C4 hydrocarbon-based radical, said radical being optionally substituted with a phenyl group.
**Rd** denotes:
- a hydrogen atom
- a linear C1-C18 or branched C3-C18 alkyl radical, or a linear or branched unsaturated C3-C18 hydrocarbon-based radical, said radical being optionally substituted with a phenyl group,
and also the cosmetically acceptable salts thereof, solvates thereof such as hydrates, and stereoisomers thereof.

2. Process according to the preceding claim, **characterized in that**:
- the compounds of formula (I) are xylose derivatives
- Y denotes a phenyl radical or a heterocycle, optionally substituted with 1 to 5 groups (ORₐ)
- X = -OR' ; (=O) ; NR_{b}R_{c}; NHOR_{d}
- R' denotes:
* a hydrogen atom,
* a linear or branched, saturated or unsaturated C1-C12, or saturated cyclic C5 or C6 alkyl radical,
* a linear or branched, saturated or unsaturated C2-C6, or saturated cyclic C5 or C6 acyl radical.
**Rₐ** denotes:
- a hydrogen atom
- a linear or branched C1-C4 alkyl radical,
- a linear or branched C2-C6 acyl radical,
when Y denotes a phenyl radical or a heterocycle substituted with 2 to 5 groups (ORₐ), two adjacent groups ORₐ may together form a divalent radical -O-CH₂-O with the proviso that when X = OH, the compound does not comprise an ethylenic double bond alpha to the carbon bearing this OH.
**R_{b}** denotes:
- a hydrogen atom
- a linear C2-C12 or branched C3-C12 alkyl radical, or a radical -CH(Z₁)-CO₂Z₂ in which
Z₁ denotes a hydrogen atom or a linear or branched C1-C6, or saturated or unsaturated cyclic C3-C6 alkyl radical, said radical being optionally substituted with at least one group chosen from =NH, -NH₂, -N(T)₂, =O,-OH, -OT, -SH, -ST, -CO₂T, phenyl, phenyl substituted with -OH or -OT, 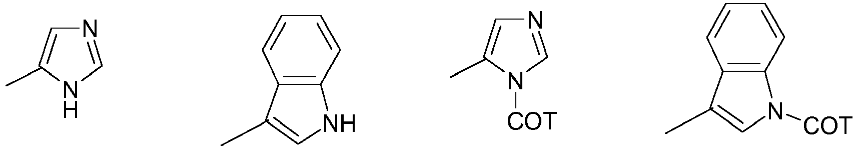 and/or interrupted with a group -NH-, -N-(COT)- or -S-with T denoting a linear C1-C6 or cyclic C5-C6 alkyl radical,
and Z₂ denotes a hydrogen atom or a linear or branched C1-C6 alkyl radical
**R_{c}** denotes:
- a hydrogen atom
- a linear C1-C4 or branched C3-C4 alkyl radical, or a linear or branched unsaturated C3-C4 hydrocarbon-based radical, said radical being optionally substituted with a phenyl group.
**Rd** denotes:
- a hydrogen atom
- a linear C1-C12 or branched C3-C12 alkyl radical, said radical being optionally substituted with a phenyl group,
and also the cosmetically acceptable salts thereof, solvates thereof such as hydrates, and stereoisomers thereof.

3. Process according to either of the preceding claims, **characterized in that**:
- the compounds of formula (I) are xylose derivatives
- Y denotes a phenyl radical or a heterocycle, optionally substituted with 1 to 3 groups (ORₐ)
- X = -OR' ; (=O) ; NR_{b}R_{c}; NHOR_{d}
- R' denotes:
* a hydrogen atom,
* a linear or branched, saturated or unsaturated C1-C4 alkyl radical,
* a linear or branched C2-C6 acyl radical.
**Rₐ** denotes:
- a hydrogen atom
- a linear or branched C1-C4 alkyl radical,
- a linear or branched C2-C6 acyl radical,
when Y denotes a phenyl radical or a heterocycle substituted with 2 or 3 groups (ORₐ), two adjacent groups ORₐ may together form a divalent radical -O-CH₂-O with the proviso that when X = OH, the compound does not comprise an ethylenic double bond alpha to the carbon bearing this OH.
**R_{b}** denotes:
- a hydrogen atom
- a linear C2-C8 or branched C3-C8 alkyl radical, or a radical -CH(Z₁)-CO₂Z₂ in which
Z₁ denotes a hydrogen atom or a linear or branched C1-C6, or saturated or unsaturated cyclic C3-C6 alkyl radical, said radical being optionally substituted with at least one group chosen from =NH, -NH₂, -N(T)₂, =O,-OH, -OT, -SH, -ST, -CO₂T, phenyl, phenyl substituted with -OH or -OT, 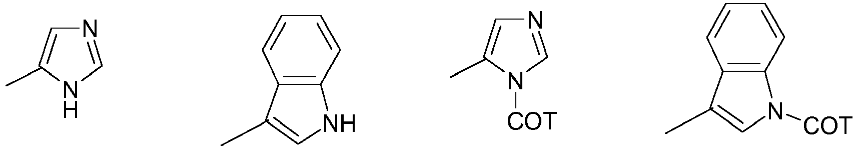 and/or interrupted with a group -NH-, -N-(COT)- or -S-with T denoting a linear C1-C6 or cyclic C5-C6 alkyl radical,
and Z₂ denotes a hydrogen atom or a linear or branched C1-C6 alkyl radical
**R_{c}** denotes:
- a hydrogen atom
- a linear C1-C4 or branched C3-C4 alkyl radical, optionally substituted with a phenyl group.
**Rd** denotes:
- a hydrogen atom
- a linear C1-C4 or branched C3-C4 alkyl radical, said radical being optionally substituted with a phenyl group,
and also the cosmetically acceptable salts thereof, solvates thereof such as hydrates, and stereoisomers thereof.

4. Process according to any one of the preceding claims, **characterized in that** the compound of formula (I) is chosen from:
**Compound 1.** : (3R,4S,5R)-2-[2-hydroxy-4-(4-hydroxy-3-methoxyphenyl)butyl]tetrahydro-2H-pyran-3,4,5-triol 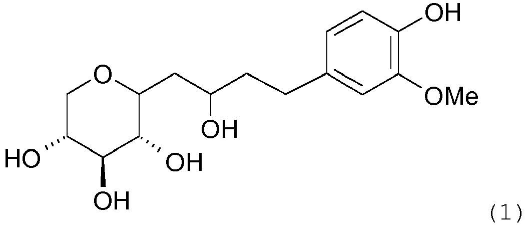
**Compound 2.** 4-(4-hydroxy-3-methoxyphenyl)-1-[(3R,4S,5R)-3,4,5-trihydroxytetrahydro-2H-pyran-2-yl]butan-2-one 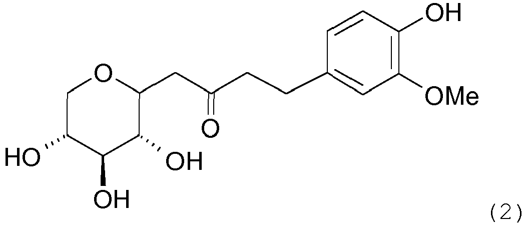
**Compound 3.** (3R,4S,5R)-2-[2-hydroxy-4-(4-hydroxyphenyl)butyl]tetrahydro-2H-pyran-3,4,5-triol 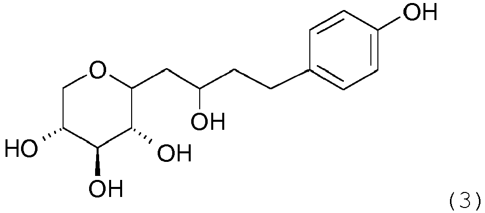
**Compound 4.** 4-(4-hydroxyphenyl)-1-[(3R,4S,5R)-3,4,5-trihydroxytetrahydro-2H-pyran-2-yl]butan-2-one 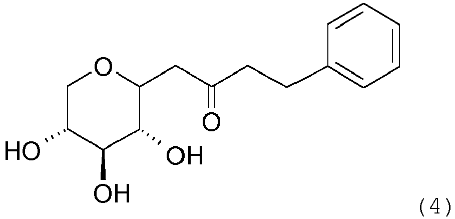
**Compound 5.** (3R,4S,5R)-2-[2-(benzylamino)-4-(4-hydroxy-3-methoxyphenyl)butyl]tetrahydro-2H-pyran-3,4,5-triol 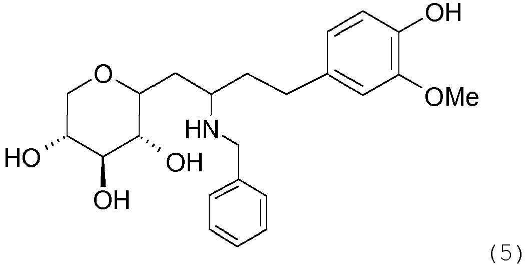
**Compound 6.** ethyl {[3-(4-hydroxy-3-methoxyphenyl)-1-{[(3R,4S,5R)-3,4,5-trihydroxytetrahydro-2H-pyran-2-yl]methyl}propyl]amino}(phenyl)acetate 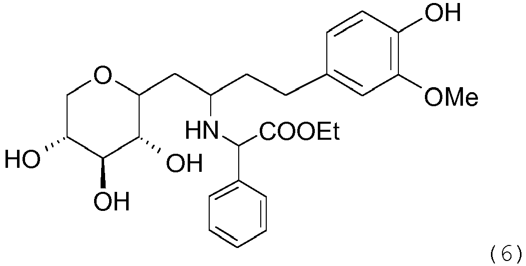
**Compound 7.** (3E)-4-phenyl-1-[(3R,4S,5R)-3,4,5-trihydroxytetrahydro-2H-pyran-2-yl]but-3-en-2-one 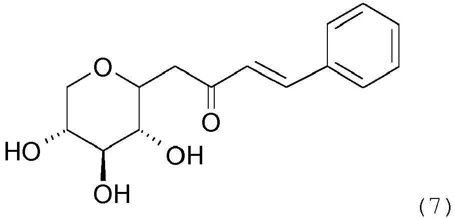
**Compound 8.** (3E)-4-(4-hydroxy-3,5-dimethoxyphenyl)-1-[(3R,4S,5R)-3,4,5-trihydroxytetrahydro-2H-pyran-2-yl]but-3-en-2-one 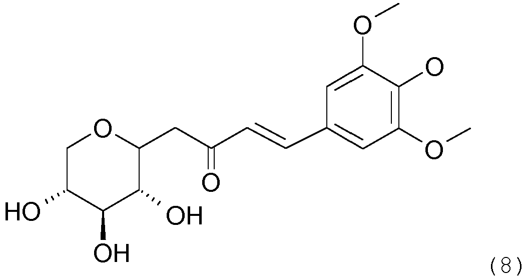 <
**Compound 9.** (3R,4S,5R)-2-[2-hydroxy-4-(2-hydroxyphenyl)butyl]tetrahydro-2H-pyran-3,4,5-triol 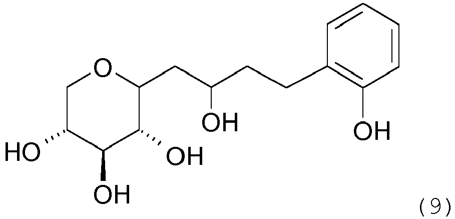
**Composé 10 :** (3R,4S,5R)-2-[2-hydroxy-4-(3-hydroxy-3-methoxyphenyl)butyl]tetrahydro-2H-pyran-3,4,5-triol 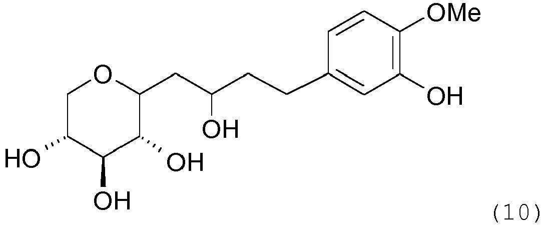
**Compound 11.** (3R,4S,5R)-2-[2-hydroxy-4-(2,4-di-hydroxyphenyl)butyl]tetrahydro-2H-pyran-3,4,5-triol 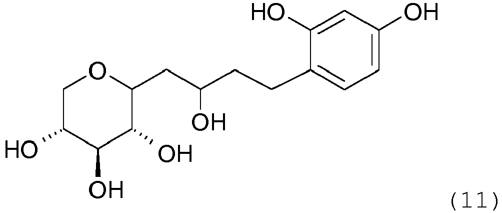
**Compound 12.** (3R,4S,5R)-2-[2-hydroxy-4-(3-ethoxy-4-hydroxyphenyl)butyl]tetrahydro-2H-pyran-3,4,5-triol 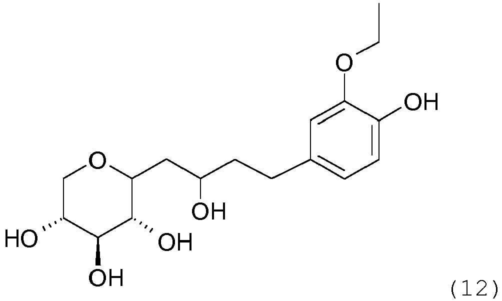
**Compound 13.** 5,9-anhydro-1,2,4-trideoxy-1-pyridin-3-yl-D-xylononitol 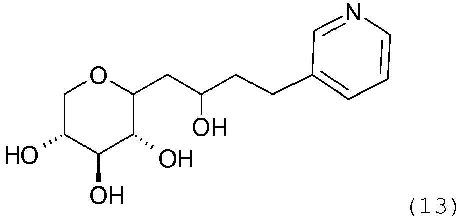
**Compound 14.** (3R,4S,5R)-2-[(2E)-2-(methoxyimino)-4-phenylbutyl]tetrahydro-2H-pyran-3,4,5-triol 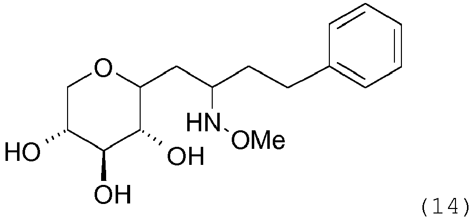
**Compound 15.** 5,9-anhydro-1,2,4-trideoxy-7-O-pentanoyl-1-phenyl-D-xylonon-3-ulose 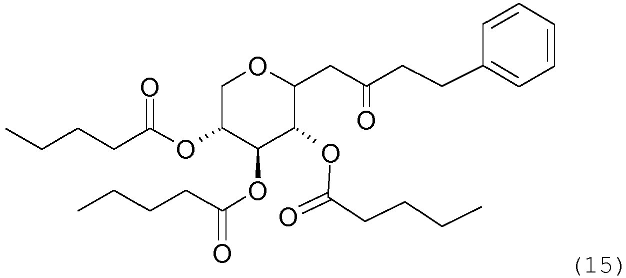
**Compound 16.** 5,9-anhydro-1,2,4-trideoxy-1-(3,4,5-trimethoxyphenyl)-D-xylononitol

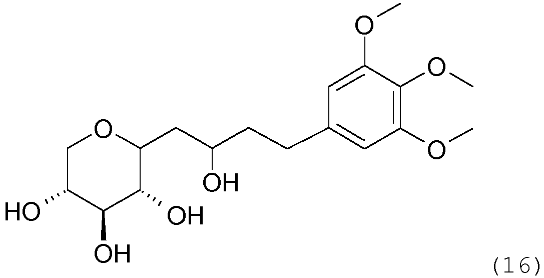

5. Process according to any one of the preceding claims, **characterized in that** the compound of formula (I) is chosen from:
**Compound 11.** (3R,4S,5R)-2-[2-hydroxy-4-(2,4-di-hydroxyphenyl)butyl]tetrahydro-2H-pyran-3,4,5-triol 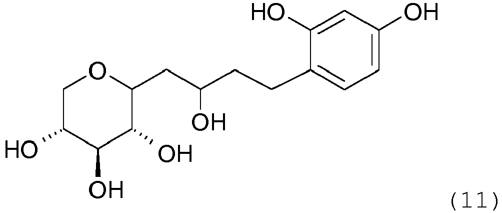
**Compound 12.** (3R,4S,5R)-2-[2-hydroxy-4-(3-ethoxy-4-hydroxyphenyl)butyl]tetrahydro-2H-pyran-3,4,5-triol

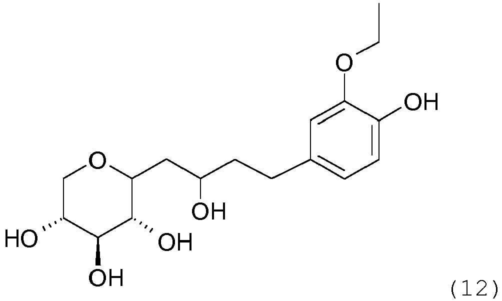

6. Process according to any one of the preceding claims, **characterized in that** the compound of formula (I) is present in an amount ranging from 0.001% to 10% by weight relative to the total weight of the composition, preferably ranging from 0.01% to 5% by weight, especially from 0.1% to 2%.

7. Cosmetic use of a compound of formula (I) as defined in any one of Claims 1 to 6, as an agent for bleaching, lightening and/or depigmenting the skin, bodily hairs or head hair.

8. C-xyloside compounds of formula (II): 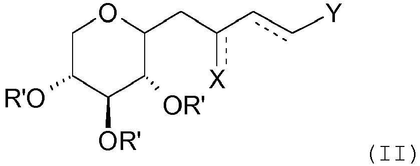 in which:
- the compounds of formula (II) are xylose derivatives
- Y denotes a heterocycle, optionally substituted with 1 to 5 groups (ORₐ)
- X = -OR' ; (=O) ; NR_{b}R_{c}; NHOR_{d}
**R'** denotes:
* a hydrogen atom,
* a saturated linear C1-C18 alkyl radical,
* an unsaturated linear C2-C18 alkyl radical,
* a saturated or unsaturated branched C3-C18 alkyl radical,
* a saturated or unsaturated cyclic C5 or C6 radical,
* a linear or branched, saturated or unsaturated C2-C18, or saturated or unsaturated cyclic C5 or C6 acyl radical.
**Rₐ** denotes:
- a hydrogen atom
- a linear or branched C1-C4 alkyl radical, or a linear or branched unsaturated C3-C4 hydrocarbon-based radical
- a linear or branched C2-C18 acyl or linear or branched C2-C18 alkenylcarbonyl radical,
**R_{b}** denotes:
- a hydrogen atom
- a linear C2-C18 or branched C3-C18 alkyl radical, or a linear or branched unsaturated C3-C4 hydrocarbon-based radical or a radical -CH(Z₁)-CO₂Z₂ in which Z₁ denotes a hydrogen atom or a linear or branched C1-C6, or saturated or unsaturated cyclic C3-C6 alkyl radical, said radical being optionally substituted with at least one group chosen from =NH, -NH₂, -N(T)₂, =O,-OH, -OT, -SH, -ST, -CO₂T, phenyl, phenyl substituted with -OH or -OT, 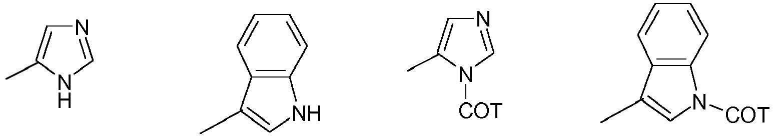
and/or interrupted with a group -NH-, -N-(COT)- or -S-with T denoting a linear or branched C1-C6 or cyclic C3-C6 alkyl radical.
and Z₂ denotes a hydrogen atom or a linear or branched C1-C6 alkyl radical
**Rc** denotes:
- a hydrogen atom
- a linear C1-C4 or branched C3-C4 alkyl radical, or a linear or branched unsaturated C3-C4 hydrocarbon-based radical, said radical being optionally substituted with a phenyl group.
**Rd** denotes:
- a hydrogen atom
- a linear C1-C18 or branched C3-C18 alkyl radical, or a linear or branched unsaturated C3-C18 hydrocarbon-based radical, said radical being optionally substituted with a phenyl group,
and also the cosmetically acceptable salts thereof, solvates thereof such as hydrates, and stereoisomers thereof.

9. C-xyloside compounds of formula (III): 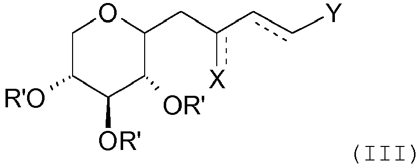 in which:
- the compounds of formula (III) are xylose derivatives
- **Y** denotes a phenyl radical, optionally substituted with 1 to 5 groups (ORₐ)
- **X** = -OR'' ; NR_{b}R_{c}; NHOR_{d}
- **R'** denotes:
* a hydrogen atom,
* a linear or branched, saturated or unsaturated C1-C18, or saturated or unsaturated cyclic C5 or C6 alkyl radical
* a linear or branched, saturated or unsaturated C1-C18, or saturated or unsaturated cyclic C5 or C6 acyl radical.
- **R"** denotes:
* a linear or branched, saturated or unsaturated C1-C18, or saturated or unsaturated cyclic C5 or C6 alkyl radical
* a linear or branched, saturated or unsaturated C1-C18, or saturated or unsaturated cyclic C5 or C6 acyl radical.
**Rₐ** denotes:
- a hydrogen atom
- a linear or branched C1-C4 alkyl radical, or a linear or branched unsaturated C3-C4 hydrocarbon-based radical
- a linear or branched C1-C18 acyl or linear or branched C2-C18 alkenylcarbonyl radical
when **Y** denotes a phenyl radical substituted with 2 to 5 groups (ORₐ), two adjacent groups ORₐ may together form a divalent radical -O-CH₂-O,
**R_{b}** denotes:
- a hydrogen atom
- a linear C2-C18 or branched C3-C18 alkyl radical, or a linear or branched unsaturated C3-C4 hydrocarbon-based radical or a radical -CH(Z₁)-CO₂Z₂ in which
Z₁ denotes a hydrogen atom or a linear or branched C1-C6, or saturated or unsaturated cyclic C3-C6 alkyl radical, said radical being optionally substituted with at least one group chosen from =NH, -NH₂, -N(T)₂, =O,-OH, -OT, -SH, -ST, -CO₂T, phenyl, phenyl substituted with -OH or -OT, 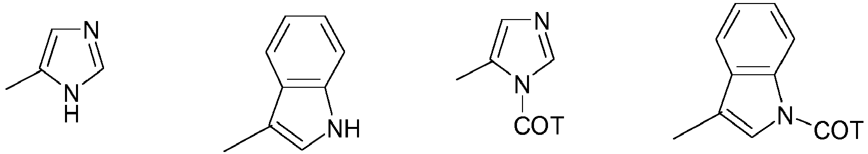 and/or interrupted with a group -NH-, -N-(COT)- or -S-with T denoting a linear or branched C1-C6 or cyclic C3-C6 alkyl radical.
and Z₂ denotes a hydrogen atom or a linear or branched C1-C6 alkyl radical
**R_{c}** denotes:
- a hydrogen atom
- a linear C1-C4 or branched C3-C4 alkyl radical, or a linear or branched unsaturated C3-C4 hydrocarbon-based radical, said radical being optionally substituted with a phenyl group.
**Rd** denotes:
- a hydrogen atom
- a linear C1-C18 or branched C3-C18 alkyl radical, or a linear or branched unsaturated C3-C18 hydrocarbon-based radical, said radical being optionally substituted with a phenyl group
and also the cosmetically acceptable salts thereof, solvates thereof such as hydrates, and stereoisomers thereof.

10. C-xyloside compounds of formula (IV): 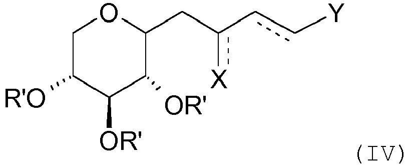 in which:
- the compounds of formula (IV) are xylose derivatives
- **Y** denotes a phenyl radical, optionally substituted with 1 to 5 groups (ORₐ)
- **X** = -OH; (=0)
- **R'** denotes:
* a linear or branched, saturated or unsaturated C1-C18, or saturated or unsaturated cyclic C5 or C6 alkyl radical
* a linear or branched, saturated or unsaturated C1-C18, or saturated or unsaturated cyclic C5 or C6 acyl radical
**Rₐ** denotes:
- a hydrogen atom
- a linear or branched C1-C4 alkyl radical, or a linear or branched unsaturated C3-C4 hydrocarbon-based radical
- a linear or branched C1-C18 acyl or linear or branched C2-C18 alkenylcarbonyl radical
when **Y** denotes a phenyl radical substituted with 2 to 5 groups (ORₐ), two adjacent groups ORₐ may together form a divalent radical -O-CH₂-O,
with the proviso that when X = OH, the compound does not comprise an ethylenic double bond alpha to the carbon bearing this OH,
and also the cosmetically acceptable salts thereof, solvates thereof such as hydrates, and stereoisomers thereof,
with the exception of the following compounds:

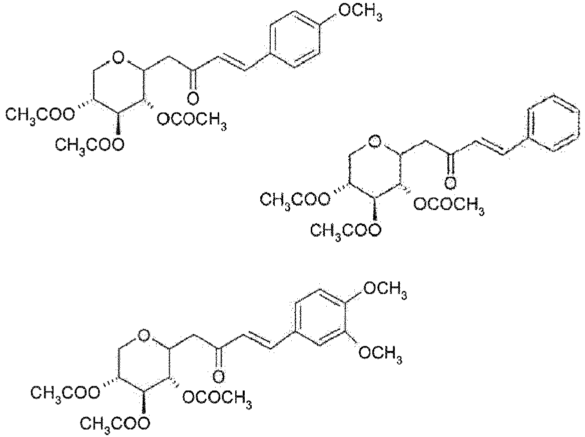

11. C-xyloside compounds of formula (V): 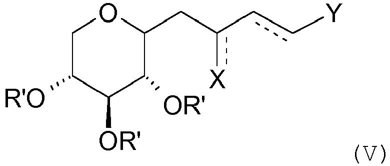 in which:
- the compounds of formula (V) are xylose derivatives
- **Y** denotes a phenyl radical, substituted with 1 to 5 groups (ORₐ)
- **X** = -OH; (=0)
- **R'** denotes a hydrogen atom
**Rₐ** denotes:
- a linear or branched C1-C4 alkyl radical, or a linear or branched unsaturated C3-C4 hydrocarbon-based radical
- a linear C1-C18 or branched C3-C18 acyl or linear or branched C2-C18 alkenylcarbonyl radical
with the proviso that when X = OH, the compound does not comprise an ethylenic double bond alpha to the carbon bearing this OH.
when **Y** denotes a phenyl radical substituted with 2 to 5 groups (ORₐ), two adjacent groups ORₐ may together form a divalent radical -O-CH₂-O,
and also the cosmetically acceptable salts thereof, solvates thereof such as hydrates, and stereoisomers thereof,
with the exception of the following compound:

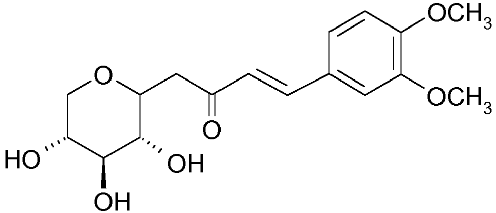

12. C-xyloside compounds of formula 5, 6, 10, 11, 12, 13, 14, 15 or 16
**Compound 5.** (3R,4S,5R)-2-[2-(benzylamino)-4-(4-hydroxy-3-methoxyphenyl)butyl]tetrahydro-2H-pyran-3,4,5-triol 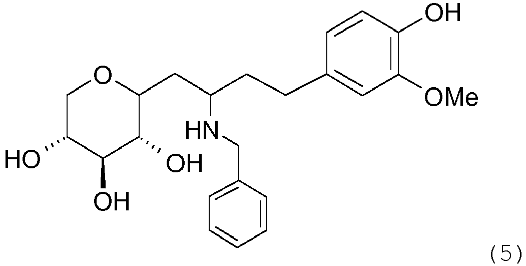
**Compound 6:** ethyl {[3-(4-hydroxy-3-methoxyphenyl)-1-{[(3R,4S,5R)-3,4,5-trihydroxytetrahydro-2H-pyran-2-yl]methyl}propyl]amino}(phenyl)acetate 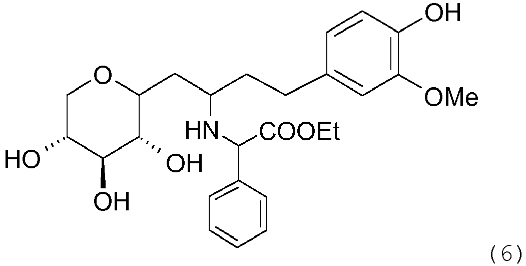
**Compound 10:** (3R,4S,5R)-2-[2-hydroxy-4-(3-hydroxy-4-methoxyphenyl)butyl]tetrahydro-2H-pyran-3,4,5-triol 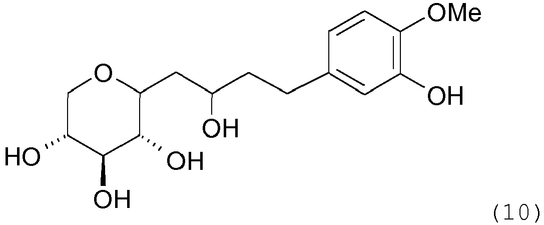
**Compound 11.** (3R,4S,5R)-2-[2-hydroxy-4-(2,4-dihydroxyphenyl)butyl]tetrahydro-2H-pyran-3,4,5-triol 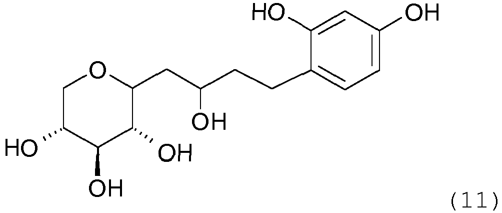
**Compound 12.** (3R,4S,5R)-2-[2-hydroxy-4-(3-ethoxy-4-hydroxyphenyl)butyl]tetrahydro-2H-pyran-3,4,5-triol 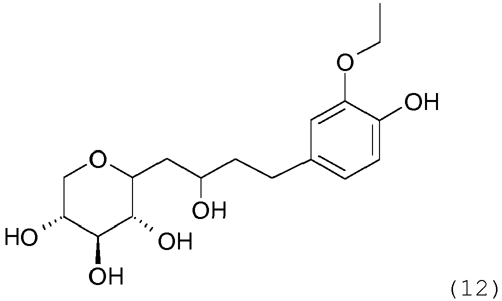
**Compound 13.** 5,9-anhydro-1,2,4-trideoxy-1-pyridin-3-yl-D-xylononitol 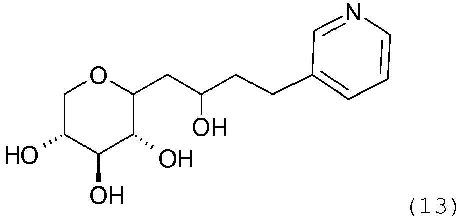
**Compound 14.** (3R,4S,5R)-2-[(2E)-2-(methoxyimino)-4-phenylbutyl]tetrahydro-2H-pyran-3,4,5-triol 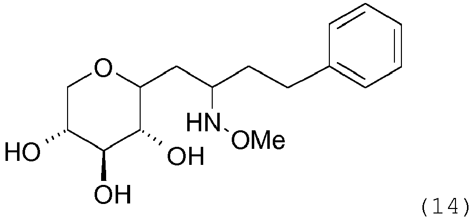
**Compound 15.** 5,9-anhydro-1,2,4-trideoxy-7-O-pentanoyl-1-phenyl-D-xylonon-3-ulose 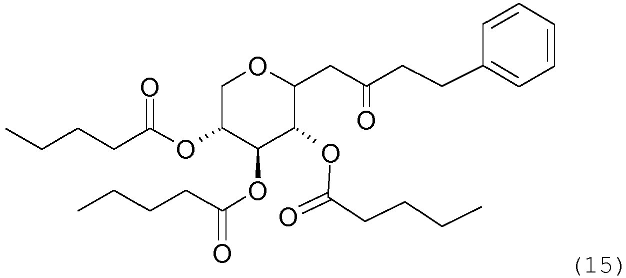
**Compound 16.** 5,9-anhydro-1,2,4-trideoxy-1-(3,4,5-trimethoxyphenyl)-D-xylononitol

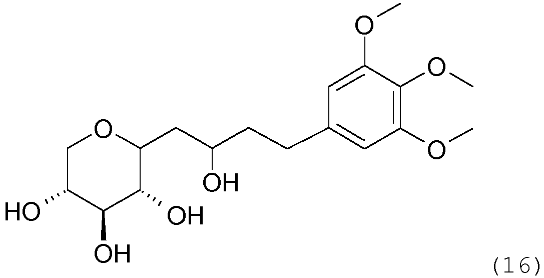

13. Composition comprising, in a physiologically acceptable medium, at least one C-xyloside compound of formula (II) according to Claim 8.

14. Composition comprising, in a physiologically acceptable medium, at least one C-xyloside compound of formula (III) according to Claim 9.

15. Composition comprising, in a physiologically acceptable medium, at least one C-xyloside compound of formula (IV) according to Claim 10.

16. Composition comprising, in a physiologically acceptable medium, at least one C-xyloside compound of formula (V) according to Claim 11.

17. Composition comprising, in a physiologically acceptable medium, at least one C-xyloside compound according to Claim 12.
